# EUROPEAN PATENT APPLICATION

(11) **EP 4 116 426 A1**
(43) Date of publication of application: **11.01.2023**
(21) Application number: 21764356.8
(22) Date of filing: 04.03.2021
(51) Int. Cl.: C12N 15/82, C12N 15/113, C12N 9/22, A01H 6/46

(54) **MULTIPLEX GENOME EDITING METHOD AND SYSTEM**

(30) Priority: 04.03.2020 CN 202010143643
(71) Applicant: Suzhou Qi Biodesign biotechnology Company Limited, Jiangsu 215127 (CN)
(72) Inventor: GAO, Caixia, Beijing 100101 (CN); LI, Chao, Beijing 100101 (CN); CHEN, Kunling, Beijing 100101 (CN)
(74) Representative: Michalski Hüttermann & Partner Patentanwälte mbB
(86) International application number: PCT/CN2021/079087
(87) International publication number: WO 2021/175289

(57) **Abstract**

The invention relates to the field of plant genetic engineering. In particular, the invention relates to a method and system for multiplex genome editing suitable for plants, especially crops. More particularly, the invention relates to a CRISPR nickase-based system and method, which can simultaneously carry out different types of genome editing.

## Description

### Technical Field

The invention relates to the field of plant genetic engineering. In particular, the invention relates to a method and system for multiplex genome editing suitable for plants, especially crops. More particularly, the invention relates to a CRISPR nickase-based system and method, which can simultaneously carry out different types of genome editing.

### Background Art

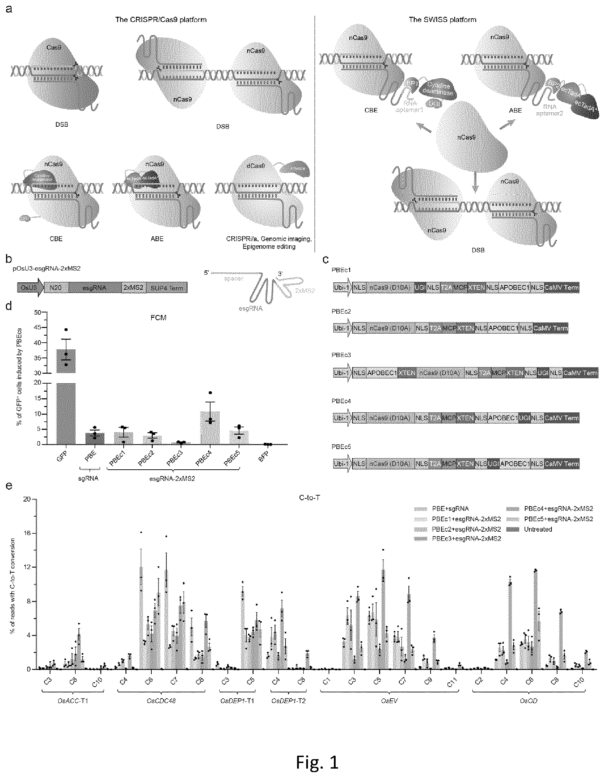

As a programmable molecular biology technology, Clustered Regularly Interspaced Short Palindromic Repeats/CRISPR-associated (CRISPR-associated, CRISPR/CAS) have greatly promoted the development of molecular biology. In the Class 2 system, more and more Cas proteins have been found and engineered, including Cas9 targeting DNA, Cas12 targeting single strand DNA (ssDNA) and RNA, Cas13 targeting RNA, and the CAST system for DNA insertion. The CRISPR/Cas system has become a super molecular toolbox due to its diversity and simplicity. In addition, Cas proteins can also be modified into variants with loss of nuclease activity. The Cas9 protein from Streptococcus pyogenes (SpCas9) consists of two nuclease domains, RuvC and HNH, which cleave the non-targeted chain and a targeted chain, respectively. Therefore, SpCas9 may be engineered into a nickase nCas9 (Nickase Cas9) by replacing aspartic acid at position 10 (Asp10) or histidine at position 840 (His840) with alanine (Ala); or Asp10 and His840 are replaced with alanine simultaneously, so that SpCas9 may lose nuclease activity to form dCas9 (Deactive Cas9). The development of these variants has promoted the CRISPR/Cas9 system to become the toolbox of genome editing system (Figure 1a). Cas9 is used to generate a double strand break (DSB) in a genome; paired nCas9s can also be used to generate a highly specific DSB on a genome, and nCas9 (D10A) is also used for the development of single-base editing systems CBE (Cytosine Base Editor) and ABE (Adenine Base Editor); dCas9 is often used to fuse with various effector proteins to achieve CRISPR interference (CRISPRi), CRISPR activation (CRISPRa), genome imaging, epigenetic modification, and the like. However, in most cases, these systems perform just one type of genome editing through one transformation.

In order to implement multiplex integrated programmable genome editing applications, several strategies have been developed so far. One strategy is to use a truncated sgRNA or crRNA to control the activity of Cas9 or Cas12a nuclease to regulate gene expression, meanwhile using a full-length sgRNA or crRNA to generate DSB at another site. Another strategy is to incorporate RNA aptamer hairpins onto the sgRNA skeleton to form a scaffold RNA (scRNA), through which the dCas9/scRNA complex can recruit gene activation or inhibition factors, to realize a dual function of activation and inhibition of gene transcription at different sites simultaneously. Still another strategy is to use multiple cognate CRISPR systems to realize a triple function of gene activation, inhibition and deletion at different target sites simultaneously. However, these multiplex strategies for genome engineering are mostly developed in bacteria, yeast and human cells. Limited by delivery methods and PAM, it is still challenging to develop a multiplex genome editing system in plants using different cognate CRISPR systems. In addition, the efficiency of homologous recombination (HR) in plants is still relatively low, so it is of great significance for breeders to stack agronomic key traits or change the gene regulation network at the genetic level. Therefore, there is an urgent need in the field for a method and system that can carry out multiplex genome editing in plants, such as crops.

### Summary of the Invention

Compared with Cas9 and dCas9, the potential of nCas9 for multiplex genome editing has not been fully exploited. The present invention provides a nCas9 nuclease-based multiplex genome editing system, which is denominated as Simultaneous and Wide-editing Induced by Single System (SWISS) (Fig. 1a). SWISS employs two kinds of scRNAs containing different RNA aptamers to recruit corresponding RNA aptamer-binding proteins fused with cytosine deaminase or adenine deaminase, which, after one transformation, can carry out two types of editing: CBE and ABE at different target sites, respectively. Another introduction of paired sgRNAs into the SWISS system may generate DSB at a third target site, thus making SWISS a CRISPR system with a triple editing function (Figure 1a).

### Brief Description of the Drawings

Fig. 1. Optimization of plant cytosine base editor constructs using RNA aptamer recruitment. (a) A nCas9 nuclease-based CRISPR scaffold RNA-programmable multiplex genome editing system. (b) The structure of pOsU3-esgRNA-2×MS2 construct with two MS2 hairpins at the 3'-end of esgRNA. (c) Structures of PBEc1 to PBEc5. Abbreviation: XTEN, 16-aa linker; NLS, nuclear localization signal; CaMV, cauliflower mosaic virus; Term, terminator. (d) Comparison of C-to-T conversions using the BFP-to-GFP reporter system induced by PBE and five PBEcs in rice protoplasts (n=3). A value with an error bar represents mean ± s.e.m. for three independent experiments, (e) Comparison of C-to-T editing frequencies of rice endogenous genes induced by PBE and five PBEcs (n=3). An untreated protoplast sample was used as control. A value with an error bar represents mean ± s.e.m. for three independent experiments.

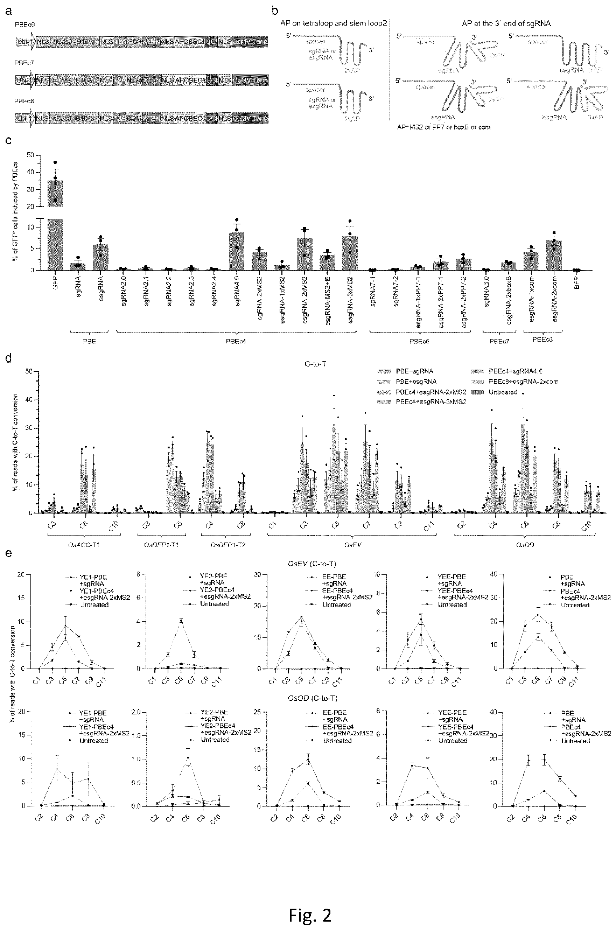

Fig. 2. Various scaffold RNAs and binding protein orthologs can effectively mediate the C-to-T conversion. (a) Structures of PBEc6 to PBEc8. (b) A schematic diagram of scRNAs with MS2, PP7, boxB or com RNA hairpins at the tetraloop and stem loop 2 or the 3'-end of sgRNA and esgRNA. (c) Comparison of C-to-T conversions using a BFP-to-GFP reporter system induced by various scRNAs and their cognate PBEcs in rice protoplasts (n=3). A value with an error bar represents mean ± s.e.m. for three independent experiments, (d) Comparison of C-to-T editing frequencies of rice endogenous genes induced by four scRNAs and their cognate PBEcs (n=3). An untreated protoplast sample was used as control. A value with an error bar represents mean ± s.e.m. for three independent experiments, (e) the C-to-T editing frequency of APOBEC1 narrow-window variants can be enhanced by employing the scRNA recruitment strategy (n=3). An untreated protoplast sample was used as control. A value with an error bar represents mean ± s.e.m. for three independent experiments.

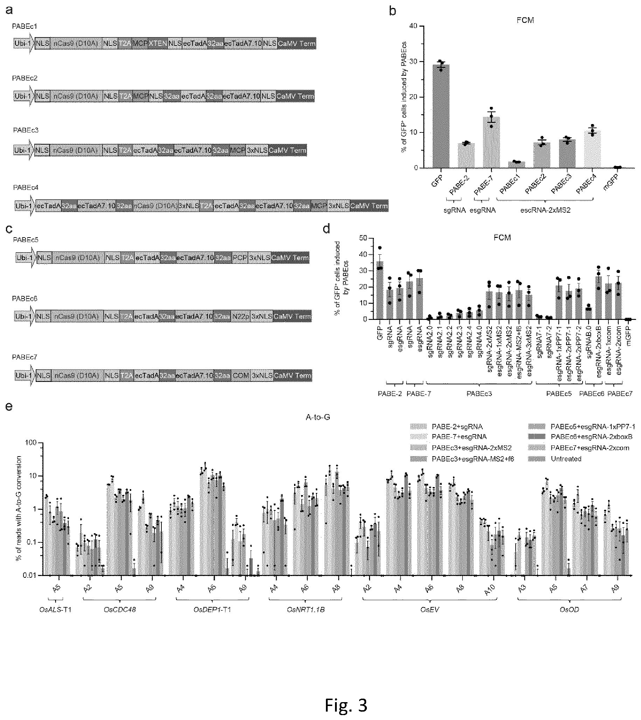

Fig. 3 Optimization of plant adenine base editor constructs using multiple scaffold RNAs and binding protein homologs. (a) Structures of PABEc1 to PABEc4. Abbreviation: ecTadA7.10, evolved Escherichia coli (E. coli) TadA; aa, amino acid; XTEN, a 16-aa linker; NLS, nuclear localization signal; CaMV, cauliflower mosaic virus; Term, terminator. (d) Comparison of A-to-G conversions using a mGFP-to-GFP reporter system induced by PABE and four PABEcs in rice protoplasts (n=3). A value with an error bar represents mean ± s.e.m. for three independent experiments, (c) Structures of PABEc5 to PABEc7. (d) Comparison of A-to-G conversions using the mGFP-to-GFP reporter system induced by various scRNAs and their cognate PBEcs in rice protoplasts (n=3). A value with an error bar represents mean ± s.e.m. for three independent experiments, (e) Comparison of A-to-G editing frequencies of rice endogenous genes induced by five scRNAs and their cognate PABEcs (n=3). An untreated protoplast sample was used as control. A value with an error bar represents mean ± s.e.m. for three independent experiments.

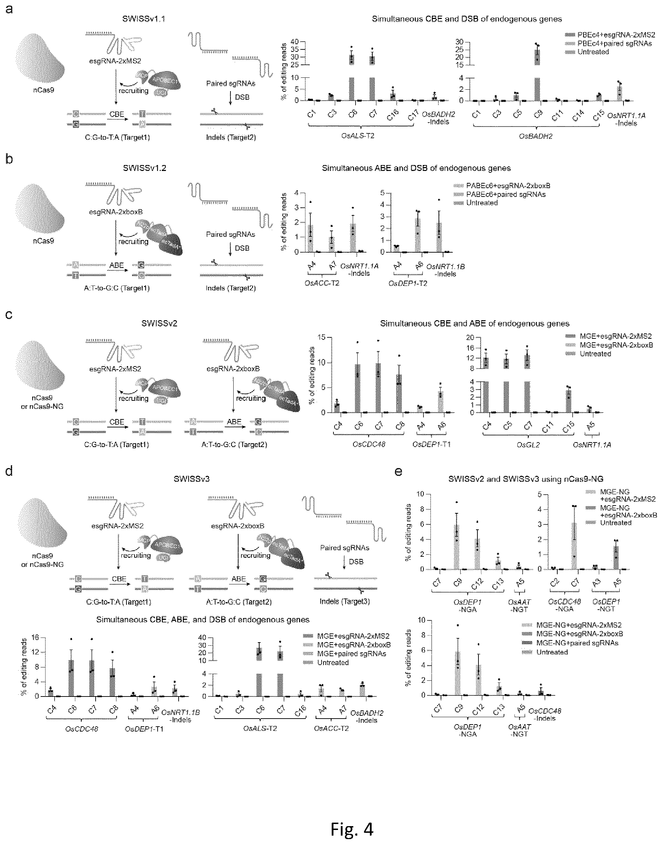

Fig. 4 nCas9 (D10A) platform-based CRISPR scaffold RNA-programmable simultaneous multiplex genome editing in rice protoplasts. (a) Simultaneous CBE and DSB induced with esgRNA-2×MS2 and paired sgRNAs by PBEc4. The left panel is the schematic diagram of strategy SWISSv1.1. On the right panel, two sets of sgRNAs are tested (n=3). A CBE target with esgRNA-2×MS2 and paired sgRNAs for DSB are assembled in the same vector. An untreated protoplast sample was used as control. A value with an error bar represents mean ± s.e.m. for three independent experiments, (b) Simultaneous ABE and DSB induced with esgRNA-2×boxB and paired sgRNAs by PABEc6. The left panel is the schematic diagram of strategy SWISSv1.2. On the right panel, two sets of sgRNAs are tested (n=3). An ABE target with esgRNA-2xboxB and paired sgRNAs for DSB were assembled in the same vector. An untreated protoplast sample was used as control. A value with an error bar represents mean ± s.e.m. for three independent experiments, (c) Simultaneous CBE and ABE induced with esgRNA-2×MS2 and esgRNA-2×boxB by MGE. The left panel is the schematic diagram of strategy SWISSv2. On the right panel, two sets of sgRNAs were tested (n=3). A CBE target with esgRNA-2×MS2 and an ABE target with esgRNA-2×boxB were assembled in the same vector. An untreated protoplast sample was used as control. A value with an error bar represents mean ± s.e.m. for three independent experiments, (c) Simultaneous CBE, ABE and DSB induced with esgRNA-2×MS2, esgRNA-2×boxB, and paired sgRNAs by MGE. The upper panel is the schematic diagram of strategy SWISSv3. In the bottom panel, two sets of sgRNAs were tested (n=3). A CBE target with esgRNA-2×MS2,an ABE target with esgRNA-2×boxB, and paired sgRNAs for DSB were assembled in the same vector. An untreated protoplast sample was used as control. A value with an error bar represents mean ± s.e.m. for three independent experiments. (e) The scope of multiplex genome editing strategies of SWISSv2 and SWISSv3 could be expanded by nCas9-NG PAM variants. Two sets of sgRNAs for SWISSv2 and one set of sgRNA for SWISSv3 are tested (n=3). Multiple sgRNAs were assembled in the same vector. An untreated protoplast sample was used as control. A value with an error bar represents mean ± s.e.m. for three independent experiments.

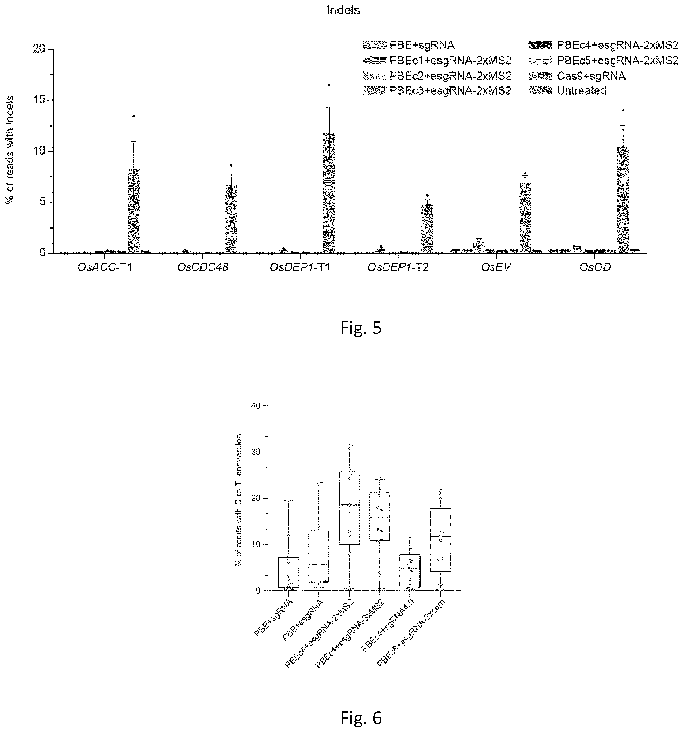

Fig. 5. Insertion/deletion (Indel) efficiencies of PBEc and Cas9 in rice protoplasts. Comparison of indel efficiencies of PBE+sgRNA, PBEc1+esgRNA-2×MS2, PBEc2+esgRNA-2×MS2, PBEc3+esgRNA-2×MS2, PBEc4+esgRNA-2×MS2, PBEc5+esgRNA-2×MS2 and Cas9+sgRNA in rice protoplasts. A value with an error bar represents mean ± s.e.m. for three independent biological replicates.
Fig. 6. Base editing efficiencies of endogenous genes obtained by different scRNAs and cognate PBEcs in rice protoplasts. The data is shown in the form of boxplot (a center line, median, border limit, the 25th and 75th percentiles of data; the upper and bottom dotted lines extend to the minimum or maximum value, respectively). The data in each boxplot includes three independent experiments (n=39).

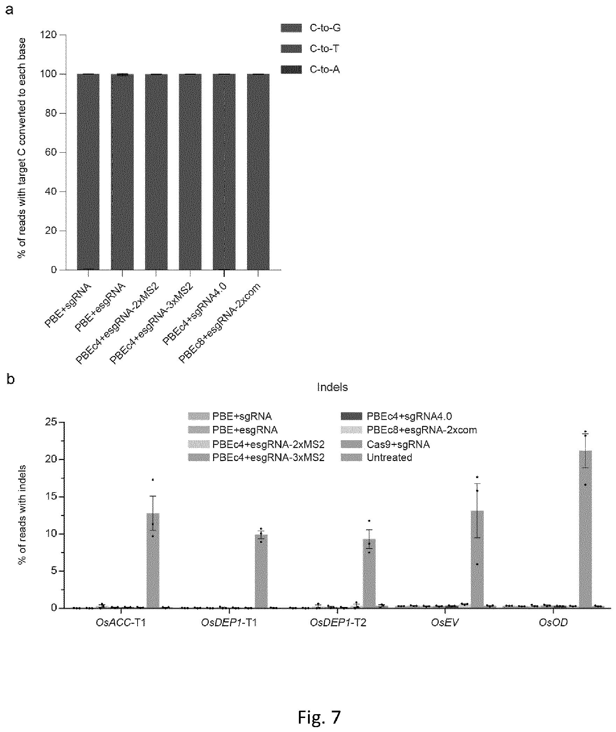

Fig. 7. Product purity and Indel frequencies yielded by esgRNA-2×MS2, esgRNA-3×MS2, sgRNA4.0 and esgRNA-2×com and related PBEcs in rice protoplasts. (a) shows the product distributions among the DNA sequencing reads edited by esgRNA-2×MS2,esgRNA-3×MS2, sgRNA4.0 and esgRNA-2×com with their cognate PBEcs in rice protoplasts. A value with an error bar represents mean ± s.e.m. for three independent experiments. (b) Indel efficiencies of esgRNA-2×MS2, esgRNA-3×MS2, sgRNA4.0 and esgRNA-2×com with their cognate PBEcs in rice protoplasts. A value with an error bar represents mean ± s.e.m. for three independent biological replicates.

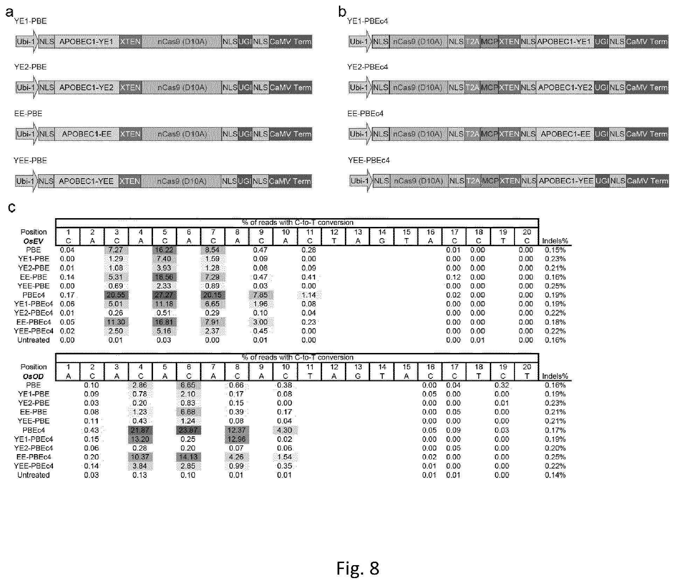

Fig. 8 C-to-T editing frequencies of APOBEC1 narrow-window variants recruited by scaffold RNAs in rice protoplasts. (a) Structures of YE1-PBE, YE2-PBE, EE-PBE and YEE-PBE. Abbreviation: XTEN, 16-aa linker; NLS, nuclear localization signal; CaMV, cauliflower mosaic virus; Term, terminator. (b) Structures of YE1-PBEc4, YE2-PBEc4, EE-PBEc4 and YEE-PBEc4. Abbreviation: XTEN, 16-aa linker; NLS, nuclear localization signal; CaMV, cauliflower mosaic virus; Term, terminator. (c) Activities of APOBEC1 narrow-window variants in the nCas9 fusion structure and in the scRNA recruitment structure. *OsEV* and *OsOD* were tested. One of three independent biological replicates are showed.

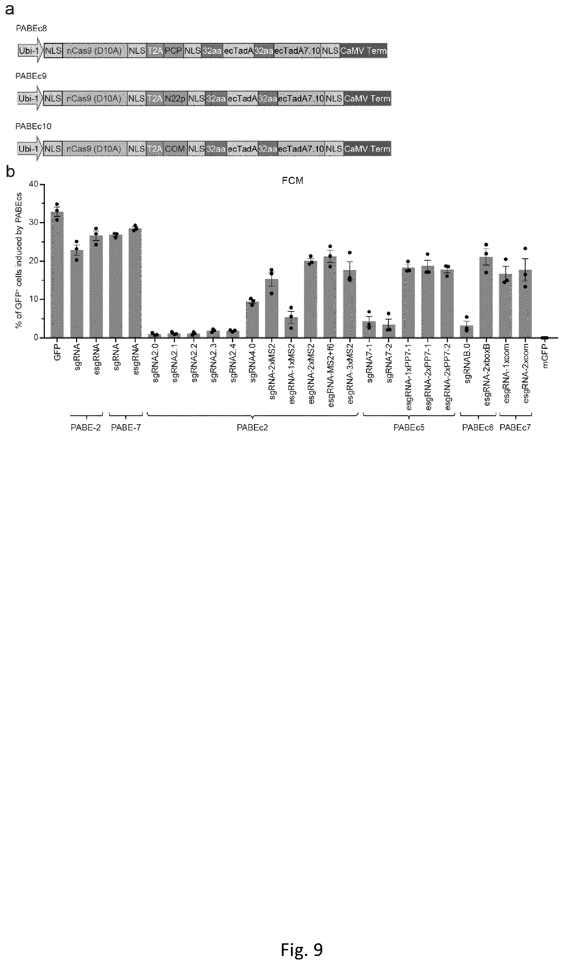

Fig. 9. Activities of PABEc8 to PABEc10 with binding proteins with adenosine deaminase at N-terminal. (a) Structures of PABEc8 to PABEc10. Abbreviation: ecTadA7.10, evolved E. coli TadA; aa, amino acid; XTEN, 16-aa linker; NLS, nuclear localization signal; CaMV, cauliflower mosaic virus; Term, terminator, (b) Comparison of A-to-G conversions using the mGFP-to-GFP reporter system induced by various scRNAs and their cognate PBEcs in rice protoplasts (n=3). A value with an error bar represents mean ± s.e.m. for three independent experiments.

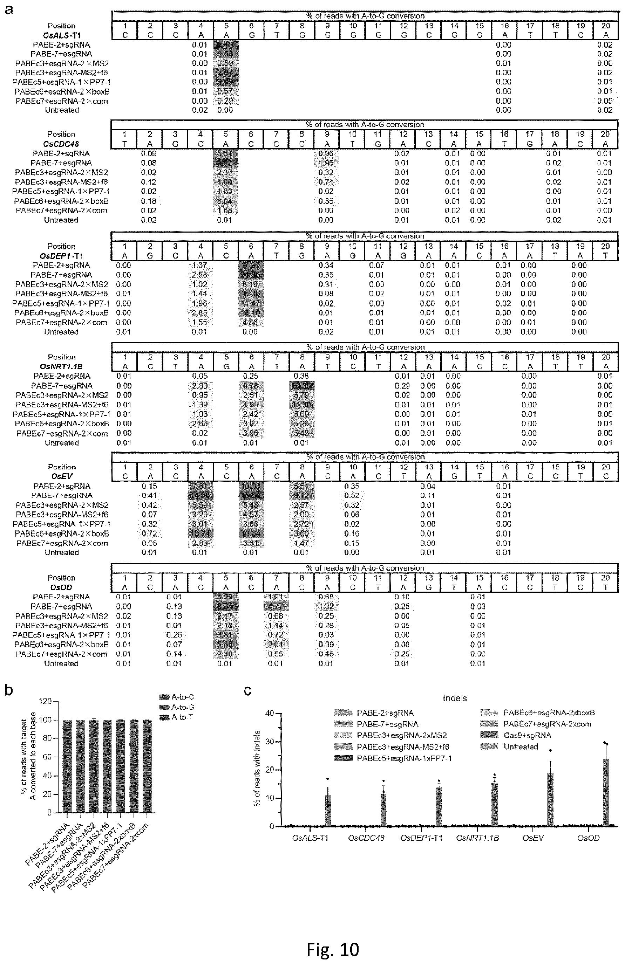

Fig. 10. Activities, product purities and indel frequencies of selected scaffold RNAs and cognate PABEcs in rice protoplasts. (a) Activities of esgRNA-2×MS2, esgRNA-MS2+f6, esgRNA-1×PP7-1, esgRNA-2×boxB and esgRNA-2×com and their cognate PABEcs in rice protoplasts. OsALS-T1, OsCDC48, OsDEP1-T1, OsNRT1.1B, OsEV and OsOD targets were tested. One of three independent biological replicates are showed. (b) shows the product distributions in the DNA sequencing read segments edited by esgRNA-2×MS2,esgRNA-MS2+f6, esgRNA-1×PP7-1, esgRNA-2×boxB and esgRNA-2×com and their related PABEcs in rice protoplasts. A value with an error bar represents mean ± s.e.m. for three independent experiments. (c) Indel efficiencies of esgRNA-2×MS2,esgRNA-MS2+f6, esgRNA-1×PP7-1, esgRNA-2×boxB and esgRNA-2×com and their cognate PABEcs in rice protoplasts. A value with an error bar represents mean ± s.e.m. for three independent biological replicates.

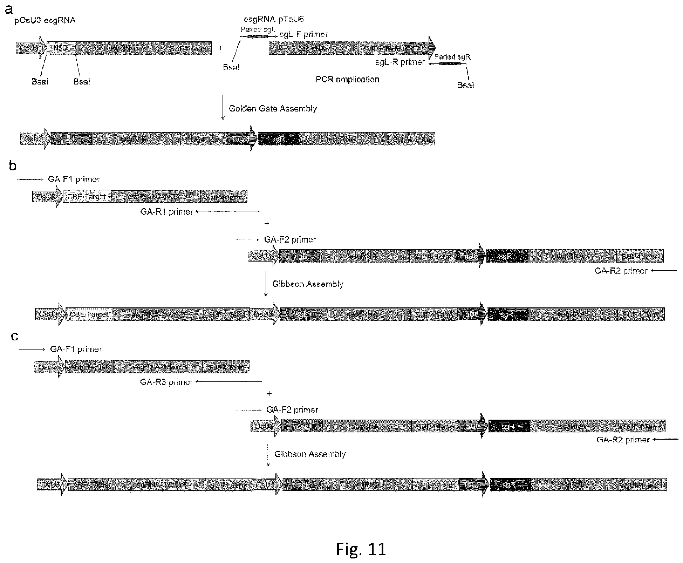

Fig. 11 shows assembly diagrams of various sgRNAs in SWISSv1.1 and SWISSv1.2. (a) An assembly diagram of paired sgRNAs. Paired sgRNAs (paired sgL and sgR) were designed in a PAM-out orientation with the distance between nicking sites of 40-68 bp. PCR products were amplified from an esgRNA-pTaU6 template, which were then inserted into BsaI site of pOsU3-esgRNA by Golden Gate Assembly. (b) Schematic assembly diagram of CBE target and paired sgRNAs. The CBE target was inserted into the BsaI site of pOsU3-esgRNA-2×MS2, and then the portion of pOsU3-CBE target-esgRNA-2×MS2 was amplified. PCR products with paired sgRNAs were amplified from paired sgRNA plasmids. The above two PCR products were assembled into the skeleton of pOsU3-esgRNA digested by EcoRI and HindIII through multi-step cloning. (c) Schematic assembly diagram of ABE target and paired sgRNAs. The ABE target was inserted into BsaI site of pOsU3-esgRNA-2×boxB, and then the portion of pOsU3-ABE target-esgRNA-2×boxB was amplified. PCR products with paired sgRNAs were amplified from paired sgRNAs plasmids. The above two PCR products were assembled into the skeleton of pOsU3-esgRNA digested by EcoRI and HindIII through multiplex one-step cloning.

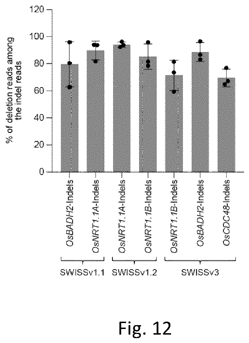

Fig. 12 shows the distributions of deletion products among indel sequencing read segments of SWISSv1.1, SWISSv1.2 and SWISSv3. A value with an error bar represents mean ± s.e.m. for three independent experiments.

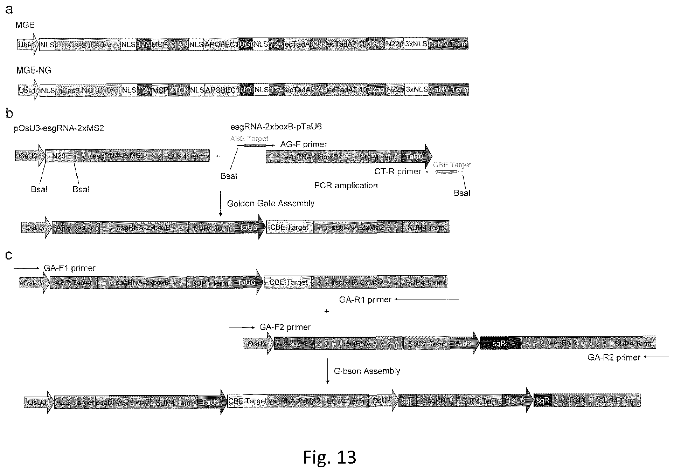

Fig. 13 shows assembly diagrams of various sgRNAs of SWISSv2 and SWISSv3. (a) Structures of MGE and MGE-NG. Abbreviation: ecTadA7.10, evolved E. coli TadA; aa, amino acid; XTEN, 16-aa linker; NLS, nuclear localization signal; CaMV, cauliflower mosaic virus; Term, terminator, (b) Schematic assembly diagram of CBE target and ABE target. PCR products were amplified from esgRNA-2×boxB-pTaU6 template, which were then inserted into the BsaI site of pOsU3-esgRNA-2×MS2 through Golden Gate Assembly. (c) Schematic assembly diagram of CBE target, ABE target and paired sgRNAs. PCR products with CBE target and ABE target were amplified from the dual-sgRNA plasmids with CBE target and ABE target. PCR products with paired sgRNAs were amplified from paired sgRNA plasmids. The above two PCR products were assembled into the skeleton of pOsU3-esgRNA digested by EcoRI and HindIII through multiplex one-step cloning.

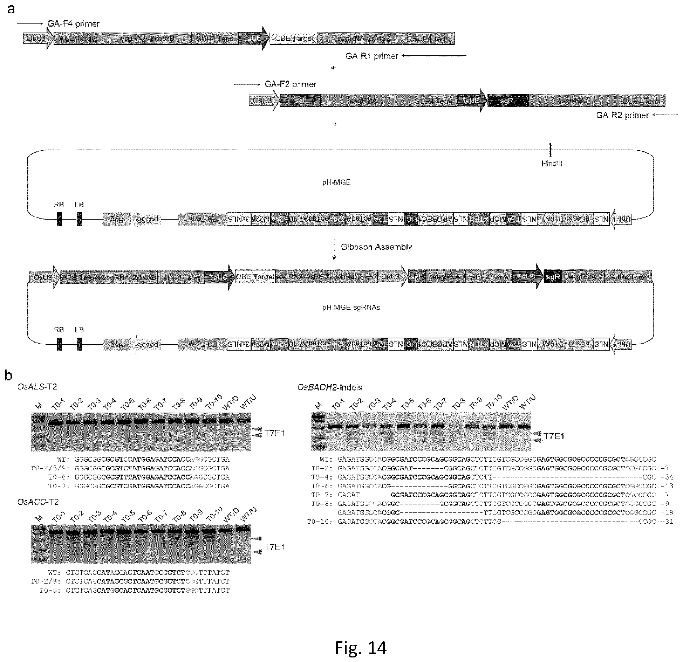

Fig. 14. Simultaneous CBE, ABE and DSB in rice plants. (a) Schematic diagram of a binary vector of CBE target, ABE target and paired sgRNAs. PCR products with CBE target and ABE target were amplified from the dual-sgRNA plasmids with CBE target and ABE target. PCR products with paired sgRNAs were amplified from paired sgRNAs plasmids. The above two PCR products were assembled into the HindIII-digested pH-MGE binary vector through multiplex one-step cloning. (b) T7E1 analysis results of OsALS-T2, OsACC-T2 and OsBADH2-Indels mutants. The target C/A base is highlighted in red. The PAM sequence is shown in brown. Ten T0 seedlings (T0-1 to T0-10) were analyzed. WT/D and WT/U indicate genomic DNA amplicon of wild-type (WT) control with or without T7E1 digestion. A total of 55 mutants were identified. The band marked by red arrow can diagnose genome editing as positive. Sequence was determined by Sanger sequencing. The indel sequencing spectrogram was analyzed by online tools DSDecodeM and TIDE.

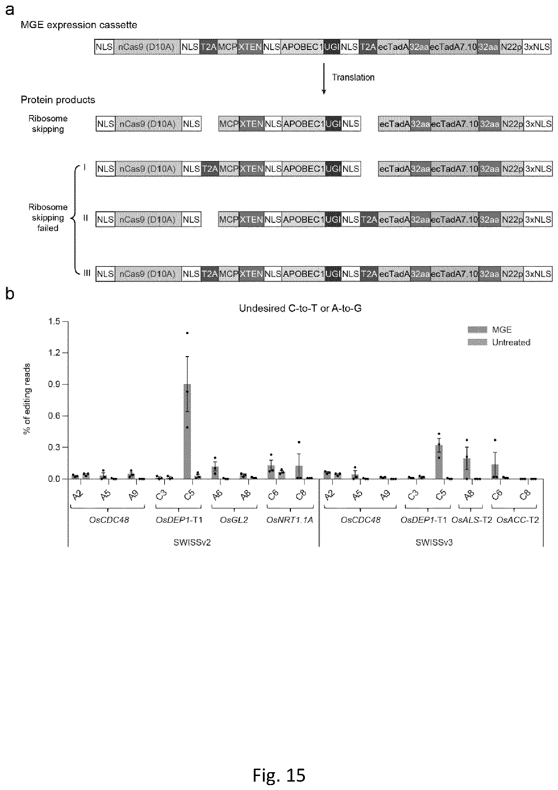

Fig. 15. Analysis of undesired off-target editing resulting from SWISSv2 and SWISSv3. (a) Schematic diagram of possible MGE protein products mediated by a "self-cleave" T2A peptide. T2A-mediated "self-cleavage" is carried out by forming a glycyl-prolyl peptide bond at the C-terminal through ribosome skipping. The position of T2A will affect the expression level of polycistronic constructs. Successful skipping can produce three independent proteins as designed. However, skipping failure may also occur, resulting in fusion protein products. Especially when MCP-APOBEC1-UGI-T2A-ecTadA-ecTadA7.10-N22p fusion protein is produced, both esgRNA-2×MS2 and esgRNA-2×boxB can recruit the fusion protein, resulting in undesired cytosine editing on ABE target and undesired adenine editing on CBE target. (b) Efficiencies of undesired off-target editing resulting from SWISSv2 and SWISSv3. Cytosine of the ABE target and adenine of the CBE target in SWISSv2 and SWISSv3 were analyzed. A value with an error bar represents mean ± s.e.m. for three independent experiments.

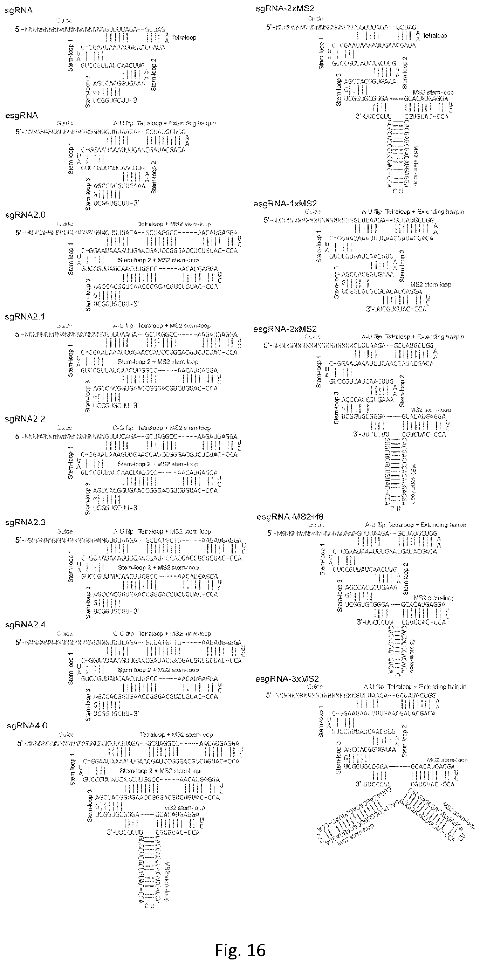

Fig. 16 shows various designed scRNA/sgRNA structures.

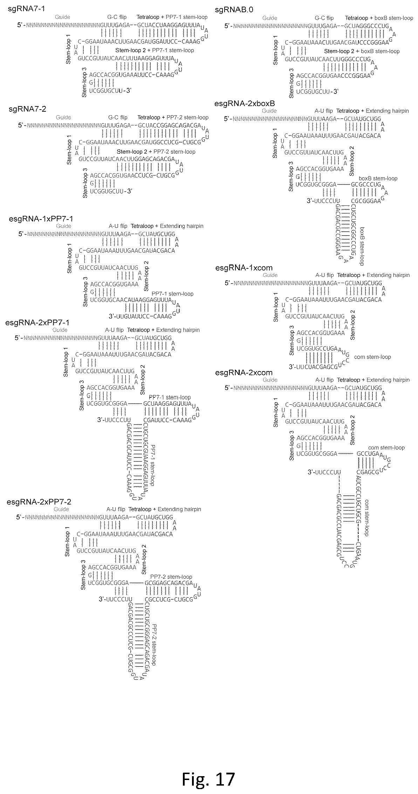

Fig. 17 shows various designed scRNA/sgRNA structures.

### Detailed Description of the Invention

### I. Definition

In the present invention, unless indicated otherwise, the scientific and technological terminologies used herein refer to meanings commonly understood by a person skilled in the art. Also, the terminologies and experimental procedures used herein relating to protein and nucleotide chemistry, molecular biology, cell and tissue cultivation, microbiology, immunology, all belong to terminologies and conventional methods generally used in the art. For example, the standard DNA recombination and molecular cloning technology used herein are well known to a person skilled in the art, and are described in details in the following references: Sambrook, J., Fritsch, E.F.and Maniatis, T., Molecular Cloning: A Laboratory Manual; Cold Spring Harbor Laboratory Press: Cold Spring Harbor, 1989. In the meantime, in order to better understand the present invention, definitions and explanations for the relevant terminologies are provided below.

As used herein, the term "and/or" encompasses all combinations of items connected by the term, and each combination should be regarded as individually listed herein. For example, "A and/or B" covers "A", "A and B", and "B". For example, "A, B, and/or C" covers "A", "B", "C", "A and B", "A and C", "B and C", and "A and B and C".

When the term "comprise" is used herein to describe the sequence of a protein or nucleic acid, the protein or nucleic acid may consist of the sequence, or may have additional amino acids or nucleotide at one or both ends of the protein or nucleic acid, but still have the activity described in this invention. In addition, those skilled in the art know that the methionine encoded by the start codon at the N-terminus of the polypeptide will be retained under certain practical conditions (for example, when expressed in a specific expression system), but does not substantially affect the function of the polypeptide. Therefore, when describing the amino acid sequence of specific polypeptide in the specification and claims of the present application, although it may not include the methionine encoded by the start codon at the N-terminus, the sequence containing the methionine is also encompassed, correspondingly, its coding nucleotide sequence may also contain a start codon; vice versa.

"Genome" as used herein encompasses not only chromosomal DNA present in the nucleus, but also organelle DNA present in the subcellular components (e.g., mitochondria, plastids) of the cell.

A "genetically modified plant" includes the plant which comprises within its genome an exogenous polynucleotide or a modified gene or expression regulatory sequence. For example, the exogenous polynucleotide is stably integrated within the genome of the plant such that the polynucleotide is passed on to successive generations. The exogenous polynucleotide may be integrated into the genome alone or as part of a recombinant DNA construct. The modified gene or expression regulatory sequence means that, in genome of the plant, said gene or sequence comprises one or more nucleotide substitution, deletion, or addition.

The term "exogenous" with respect to sequence means a sequence that originates from a foreign species, or, if from the same species, is substantially modified from its native form in composition and/or genomic locus by deliberate human intervention.

"Polynucleotide", "nucleic acid sequence", "nucleotide sequence", or "nucleic acid fragment" are used interchangeably to refer to a polymer of RNA or DNA that is single- or double-stranded, optionally containing synthetic, non-natural or altered nucleotide bases. Nucleotides (usually found in their 5'-monophosphate form) are referred to by their single letter designation as follows: "A" for adenylate or deoxyadenylate (for RNA or DNA, respectively), "C" for cytidylate or deoxycytidylate, "G" for guanylate or deoxyguanylate, "U" for uridylate, "T" for deoxythymidylate, "R" for purines (A or G), "Y" for pyrimidines (C or T), "K" for G or T, "H" for A or C or T, "I" for inosine, and "N" for any nucleotide.

"Polypeptide", "peptide", "amino acid sequence" and "protein" are used interchangeably herein to refer to a polymer of amino acid residues. The terms apply to amino acid polymers in which one or more amino acid residue is an artificial chemical analogue of a corresponding naturally occurring amino acid, as well as to naturally occurring amino acid polymers. The terms "polypeptide", "peptide", "amino acid sequence", and "protein" are also inclusive of modifications including, but not limited to, glycosylation, lipid attachment, sulfation, gamma-carboxylation of glutamic acid residues, hydroxylation and ADP-ribosylation.

As used herein, an "expression construct" refers to a vector suitable for expression of a nucleotide sequence of interest in an organism, such as a recombinant vector. "Expression" refers to the production of a functional product. For example, the expression of a nucleotide sequence may refer to transcription of the nucleotide sequence (such as transcribe to produce an mRNA or a functional RNA) and/or translation of RNA into a protein precursor or a mature protein.

"Expression construct" of the invention may be a linear nucleic acid fragment, a circular plasmid, a viral vector, or, in some embodiments, an RNA that can be translated (such as an mRNA), for example, an RNA transcribed in vitro.

"Expression construct" of the invention may comprise regulatory sequences and nucleotide sequences of interest that are derived from different sources, or regulatory sequences and nucleotide sequences of interest derived from the same source, but arranged in a manner different than that normally found in nature.

"Regulatory sequence" or "regulatory element" are used interchangeably and refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences may include, but are not limited to, promoters, translation leader sequences, introns, and polyadenylation recognition sequences.

"Promoter" refers to a nucleic acid fragment capable of controlling the transcription of another nucleic acid fragment. In some embodiments of the present invention, the promoter is a promoter capable of controlling the transcription of a gene in a cell, whether or not it is derived from the cell. The promoter may be a constitutive promoter or a tissue-specific promoter or a developmentally-regulated promoter or an inducible promoter.

"Constitutive promoter" refers to a promoter that may cause expression of a gene in most circumstances in most cell types. "Tissue-specific promoter" and "tissue-preferred promoter" are used interchangeably, and refer to a promoter that is expressed predominantly but not necessarily exclusively in one tissue or organ, but that may also be expressed in one specific cell or cell type. "Developmentally regulated promoter" refers to a promoter whose activity is determined by developmental events. "Inducible promoter" selectively expresses a DNA sequence operably linked to it in response to an endogenous or exogenous stimulus (environment, hormones, or chemical signals, and so on).

Examples of promoters that can be used in the present invention include, but are not limited to, polymerase (pol) I, pol II, or pol III promoters. Examples of pol I promoters include the chicken RNA pol I promoter. Examples of pol II promoters include, but are not limited to, the cytomegalovirus immediate early (CMV) promoter, the Rous sarcoma virus long terminal repeat (RSV-LTR) promoter, and the simian virus 40 (SV40) immediate early promoter. Examples of pol III promoters include U6 and H1 promoters. Inducible promoters such as the metallothionein promoter can be used. Other examples of promoters include T7 phage promoter, T3 phage promoter, β-galactosidase promoter, and Sp6 phage promoter. When used in plants, the promoter may be cauliflower mosaic virus 35S promoter, maize Ubi-1 promoter, wheat U6 promoter, rice U3 promoter, maize U3 promoter, rice actin promoter.

As used herein, the term "operably linked" means that a regulatory element (for example but not limited to, a promoter sequence, a transcription termination sequence, and so on) is associated to a nucleic acid sequence (such as a coding sequence or an open reading frame), such that the transcription of the nucleotide sequence is controlled and regulated by the transcriptional regulatory element. Techniques for operably linking a regulatory element region to a nucleic acid molecule are known in the art.

"Introduction" of a nucleic acid molecule (e.g., plasmid, linear nucleic acid fragment, RNA, etc.) or protein into an organism means that the nucleic acid or protein is used to transform a cell of the organism such that the nucleic acid or protein functions in the cell. As used in the present invention, "transformation" includes both stable and transient transformations. "Stable transformation" refers to the introduction of an exogenous nucleotide sequence into the genome, resulting in the stable inheritance of foreign genes. Once stably transformed, the exogenous nucleic acid sequence is stably integrated into the genome of the organism and any of its successive generations. "Transient transformation" refers to the introduction of a nucleic acid molecule or protein into a cell, performing its function without the stable inheritance of an exogenous gene. In transient transformation, the exogenous nucleic acid sequence is not integrated into the genome.

"Trait" refers to the physiological, morphological, biochemical, or physical characteristics of a cell or an organism.

"Agronomic trait" is a measurable parameter including but not limited to, leaf greenness, yield, growth rate, biomass, fresh weight at maturation, dry weight at maturation, fruit yield, seed yield, total plant nitrogen content, fruit nitrogen content, seed nitrogen content, nitrogen content in a vegetative tissue, total plant free amino acid content, fruit free amino acid content, seed free amino acid content, free amino acid content in a vegetative tissue, total plant protein content, fruit protein content, seed protein content, protein content in a vegetative tissue, drought tolerance, nitrogen uptake, root lodging, harvest index, stalk lodging, plant height, ear height, ear length, disease resistance, cold resistance, salt tolerance, and tiller number and so on.

### II. Multiplex genome editing system

In one aspect, the present invention provides a genome editing system for multiplex editing in a plant, especially a crop, comprising:
i) a CRISPR nickase and/or an expression construct containing a nucleotide sequence encoding the CRISPR nickase; and
ii) one or more or all items selected from the group consisting of:
   ii-1) a first scRNA targeting a first target region in the plant genome and/or an expression construct containing a nucleotide sequence encoding the first scRNA, wherein the first scRNA comprises at least one first RNA aptamer; and, a first fusion protein and/or an expression construct containing a nucleotide sequence encoding the first fusion protein, wherein the first fusion protein comprises a first RNA aptamer-specific binding protein and a cytosine deamination domain;
   ii-2) a second scRNA targeting a second target region in the plant genome and/or an expression construct containing a nucleotide sequence encoding the second scRNA, wherein the second scRNA comprises at least one second RNA aptamer; and, a second fusion protein and/or an expression construct containing a nucleotide sequence encoding the second fusion protein, wherein the second fusion protein comprises a second RNA aptamer-specific binding protein and an adenine deamination domain;
   ii-3) paired gRNAs targeting a third target region in the plant genome and/or an expression construct containing nucleotide sequences encoding the paired gRNAs, wherein the paired gRNAs target different strands of DNA in the third target region, respectively.

As used herein, "genome editing system" refers to the combination of components required for editing the genomes of a cell or organism. Individual components of the system, such as the CRISPR nickase, the first scRNA, the first fusion protein, the second scRNA, the second fusion protein, the paired gRNAs, and their expression vectors can exist independently of one another or in any combination as a composition.

As used herein, "CRISPR nickase" refers to the nickase form of CRISPR nuclease, which forms a nick in a double-stranded nucleic acid molecule, instead of completely cutting the double-stranded nucleic acid, and still retains the gRNA-directed sequence specific DNA binding ability.

In some embodiments, the CRISPR nickase is a Ca9 nickase, such as Cas9 nickase derived from S. pyogenes Cas9 (SpCas9). In some embodiments, the Cas9 nickase comprises the amino acid sequence shown in SEQ ID NO: 25 (nCas9 (D10A)).

In some embodiments, the Cas9 nickase is a Cas9 variant nickase capable of recognizing the PAM sequence 5'-NG-3', which comprises the amino acid sequence shown in SEQ ID NO: 48 (nCas9-NG (D10A)).

As used herein, "guide RNA" and "gRNA" are used interchangeably, and refer to RNA molecules that can form a complex with a CRISPR nuclease or its derivative protein, such as a CRISPR nickase, and can target the complex to a target sequence due to having certain identity with the target sequence. gRNA targets a target sequence through base pairing with the complementary strand of the target sequence. For example, gRNA used by a Cas9 nuclease or its derivative protein, such as a Cas9 nickase, is usually composed of crRNA and tracrRNA molecules, which are partially complementary to form a complex, wherein the crRNA comprises a guide sequence (also called spacer), which has sufficient identity with the target sequence to hybridize with the complementary strand of the target sequence and guides the CRISPR complex (Cas9+crRNA+tracrRNA) to specifically bind to the target sequence. However, it is known in the art that a single guide RNA (sgRNA) can be designed, which contains the characteristics of both crRNA and tracrRNA.

In certain embodiments, the sgRNA comprises the nucleotide sequence shown in SEQ ID NO: 3 or SEQ ID NO: 4.

As used herein, "RNA aptamer" refers to an RNA molecule that can specifically bind to a specific protein. Examples of RNA aptamers suitable for the present invention include, but are not limited to, MS2, PP7, boxB and com, and their corresponding RNA aptamer-specific binding proteins are MCP (SEQ ID NO: 34), PCP (SEQ ID NO: 35), N22p (SEQ ID NO: 36) and COM (SEQ ID NO: 37).

As used herein, "scRNA" or its interchangeable term "scaffold RNA" refers to RNA molecules formed by incorporating an RNA aptamer onto gRNA (such as sgRNA) of the CRISPR system, which retains the functions of gRNA and can recruit a specific binding protein for the RNA aptamer or a fusion protein containing the specific binding protein.

In some embodiments, the scRNA comprises two or more RNA aptamers. In some embodiments, the scRNA comprises a nucleotide sequence shown in one of SEQ ID NOs: 5 -24.

In some preferred embodiments, the first scRNA comprises the nucleotide sequence shown in SEQ ID NO: 13 or 15. Accordingly, the first RNA aptamer-specific binding protein comprises the amino acid sequence shown in SEQ ID NO: 34.

In some preferred embodiments, the first scRNA comprises the nucleotide sequence shown in SEQ ID NO: 24. Accordingly, the first RNA aptamer-specific binding protein comprises the amino acid sequence shown in SEQ ID NO: 37.

In some preferred embodiments, the second scRNA comprises the nucleotide sequence shown in SEQ ID NO: 22. Accordingly, the second RNA aptamer-specific binding protein comprises the amino acid sequence shown in SEQ ID NO: 36.

As used herein, "cytosine deamination domain" refers to a domain that can accept a single-stranded DNA as a substrate and catalyze the deamination of cytidine or deoxycytidine to uracil or deoxyuracil, respectively. In some embodiments, the cytosine deaminase domain comprises at least one (e.g., one or two) cytosine deaminase polypeptide.

In the present invention, the cytidine deamination domain in the first fusion protein enables deamination of cytidine C in a single-stranded DNA generated in the formation of a CRIPR nickase-first scRNA-first fusion protein-DNA complex to uracil U, and further C-to-T base substitution through base mismatch repair.

Examples of cytosine deaminases that can be used in the present invention include, but are not limited to, APOBEC1 deaminase, activation-induced cytidine deaminase (AID), APOBEC3G, CDA1, human APOBEC3A deaminase, or their functional variants. In some embodiments, the cytosine deaminase is APOBEC1 deaminase or its functional variant. In some embodiments, the cytosine deaminase comprises the amino acid sequence shown in one of SEQ ID NOs: 26 -30.

In some embodiments, in the first fusion protein, the first RNA aptamer-specific binding protein is located at the N-terminal of the cytosine deamination domain. In some embodiments, in the first fusion protein, the first RNA aptamer-specific binding protein is fused with the cytosine deamination domain via a linker.

In some embodiments, the first fusion protein further comprises a uracil DNA glycosylase inhibitor (UGI). In cells, the uracil DNA glycosylase catalyzes the removal of U from DNA and initiates base excision repair (BER) to repair of U: G to C: G. Therefore, without being bound by any theory, the inclusion of uracil DNA glycosylase inhibitor (UGI) in the first fusion protein of the present invention will be capable of increasing the efficiency of C-to-T base editing.

In some embodiments, the UGI comprises the amino acid sequence shown in SEQ ID NO: 31.

As used herein, "adenine deamination domain" refers to a domain that can accept a single-stranded DNA as a substrate and catalyze adenosine or deoxyadenosine (A) to form inosine (I). In some embodiments, the adenine deamination domain comprises at least one (e.g., one) DNA-dependent adenine deaminase polypeptide.

In the present invention, the adenine deamination domain in the fusion protein enables deamination of adenosine in a single-stranded DNA generated in the formation of a CRIPR nickase-second scRNA-second fusion protein-DNA complex to inosine (I), and A-to-G substitution through base mismatch repair since DNA polymerases will treat inosine (I) as guanine (G).

In some embodiments, the DNA-dependent adenine deaminase is a variant of E. coli tRNA adenine deaminase TadA (ecTadA). An exemplary wild-type ecTadA amino acid sequence is shown in SEQ ID NO: 32. In some preferred embodiments of the present invention, the DNA-dependent adenine deaminase comprises the amino acid sequence shown in SEQ ID NO: 33.

As E. coli tRNA adenine deaminase (ecTadA) usually functions as a dimer, it is expected that the dimer formed by two DNA-dependent adenine deaminases or the dimer formed by a DNA-dependent adenine deaminase and a wild-type adenine deaminase can significantly improve the A-to-G editing activity of the fusion protein. In some preferred embodiments, the adenine deamination domain comprises two DNA-dependent adenine deaminases.

In some preferred embodiments, the adenine deamination domain further comprises a corresponding wild-type adenine deaminase (e.g., E. coli tRNA adenine deaminase TadA) fused with the DNA-dependent adenine deaminase (e.g., a DNA-dependent variant of E. coli tRNA adenine deaminase TadA). In some preferred embodiments, the DNA-dependent adenine deaminase (e.g., a DNA-dependent variant of E. coli tRNA adenine deaminase TadA) is fused to the C-terminal of a corresponding wild-type adenine deaminase (e.g., E. coli tRNA adenine deaminase TadA).

In some embodiments, the fusion between the two DNA-dependent adenine deaminases (e.g., a DNA-dependent variant of E. coli tRNA adenine deaminase TadA) or between the DNA-dependent adenine deaminase (e.g., a DNA-dependent variant of E. coli tRNA adenine deaminase TadA) and the corresponding wild-type adenine deaminase (e.g., E. coli tRNA adenine deaminase TadA) is by a linker.

In some embodiments, in the second fusion protein, the second RNA aptamer-specific binding protein is located at the C-terminal of the adenine deamination domain. In some embodiments, in the second fusion protein, the second RNA aptamer-specific binding protein is fused with the adenine deamination domain via a linker.

As used herein, the "linker" may be a nonfunctional amino acid sequence having 1-50 (such as 1, 2, 3, 4, 5, 6, 7, 8, 9, 10, 11, 12, 13, 14, 15, 16, 17, 18, 19, 20 or 20-25, 25-50) or more amino acids in length, without secondary or higher structures. For example, the linker is a flexible linker. In some embodiments, the linker is 16 amino acids in length, such as comprising the amino acid sequence shown in SEQ ID No: 41. In some embodiments, the linker is 36 amino acids in length, such as comprising the amino acid sequence shown in SEQ ID NO: 42 or 43.

In some embodiments of the present invention, the CRISPR nickase, the first fusion protein and/or the second fusion protein of the present invention may also contain a nuclear localization sequence (NLS). Generally, one or more NLSs in the CRISPR nickase, the first fusion protein and/or the second fusion protein should have sufficient strength to drive the protein in the nucleus of a cell to accumulate in an amount that can realize its base editing function. Generally speaking, the strength of nuclear localization activity is determined by the number and position of NLS in the protein, one or more specific NLS used, or combinations of these factors.

In some embodiments of the present invention, NLS in the CRISPR nickase, the first fusion protein and/or the second fusion protein of the present invention may be located at the N-terminal and/or C-terminal or in the middle. In some embodiments, the CRISPR nickase, the first fusion protein and/or the second fusion protein may also contain about 1, 2, 3, 4, 5, 6, 7, 8, 9, 10 or more NLSs. In some embodiments, the CRISPR nickase, the first fusion protein and/or the second fusion protein may also contain about 1, 2, 3, 4, 5, 6, 7, 8, 9, 10 or more NLSs at or close to the N-terminal. In some embodiments, the CRISPR nickase, the first fusion protein and/or the second fusion protein may also contain about 1, 2, 3, 4, 5, 6, 7, 8, 9, 10 or more NLSs at or close to the C-terminal. When more than one NLS exist, each of which can be selected independently of one another. In certain embodiments, NLS contains the amino acid sequence shown in SEQ ID NO: 39 or 40.

In addition, based on the DNA position to be edited, the CRISPR nickase, the first fusion protein and/or the second fusion protein of the present invention may also comprise other localization sequences, such as a cytoplasmic localization sequence, a chloroplast localization sequence, and a mitochondrial localization sequence.

In some embodiments, the CRISPR nickase, the first fusion protein and/or the second fusion protein of the present invention may be interlinked by a "self-cleavage peptide".

As used herein, the "self-cleavage peptide" means a peptide that can perform self-cleavage in cells. For example, the self-cleavage peptide may contain a protease recognition site, so as to be recognized and specifically cleaved by proteases in cells. Alternatively, the self-cleavage peptide may be a 2A polypeptide. The 2A polypeptide is a class of short peptide from viruses, and its self-cleavage occurs during translation. When two different target polypeptides are linked by 2A polypeptide and expressed in the same reading frame, two target polypeptides are generated almost in a ratio of 1:1. A commonly used 2A polypeptide may be P2A from porcine techovirus-1, T2A from Thosea asigna virus, E2A from Equine Rhinitis Virus, and F2A from foot-and-mouth disease virus. A variety of functional variants of these 2A polypeptides are also known in the art, which can also be used in the present invention. In some specific embodiments, the 2A polypeptide is T2A, such as comprising the amino acid sequence shown in SEQ ID No: 38.

The CRISPR nickase, the first fusion protein and/or the second fusion protein of the present invention can be expressed in the same expression vector by the use of the self-cleavage peptide.

In some embodiments, the first scRNA, the second scRNA and/or the paired gRNAs can be expressed by the same expression construct.

By using the genome editing system of the present invention, different types of genome editing at different target sites can be carried out simultaneously through one transformation. For example, if i), ii-1), ii-2) and ii-3) in the system are co-introduced (in the same vector or in separate vectors) into a plant, C-to-T editing at the first target site, A-to-G editing at the second target site, and deletion mutation at the third target site can be carried out through one transformation.

### III. Method for generating genetically modified plants

In another aspect, the present invention provides a method for generating a genetically modified plant, such as a genetically modified crop, the method comprising introducing the genome editing system of the present invention into a plant.

In some embodiments, i) and ii-1) of the system are co-introduced into a plant, resulting in C-to-T editing at the first target site.

In some embodiments, i), ii-1) and ii-2) of the system are co-introduced into a plant, resulting in C-to-T editing at the first target site, A-to-G editing at the second target site.

In some embodiments, i), ii-2) and ii-3) of the system are co-introduced into a plant, resulting in A-to-G editing at the second target site, and deletion mutation at the third target site.

In some embodiments, i), ii-1) and ii-3) of the system are co-introduced into a plant, resulting in C-to-T editing at the first target site, and deletion mutation at the third target site.

In some embodiments, i), ii-1), ii-2) and ii-3) of the system are co-introduced into a plant, resulting in C-to-T editing at the first target site, A-to-G editing at the second target site, and deletion mutation at the third target site.

In some embodiments, i), ii-1), ii-2) and ii-3) and combinations thereof in the system are co-introduced into the plant at the same time, such as in the same vector or in one transformation.

In some embodiments, the method comprises:
a) introducing i) of the genome editing system of the invention into a plant to obtain a transgenic plant stably expressing the CRSPR nickase;
b) introducing i-1), ii-2) or ii-3) or any combination thereof of the genome editing system of the invention into the transgenic plant obtained in step a).

In the method of the present invention, the genome editing system can be introduced into a plant by various methods well known to those skilled in the art. Methods that can be used to introduce a genome editing system of the invention into a plant include, but are not limited to, gene gun method, PEG-mediated protoplast transformation, Agrobacterium-mediated transformation, plant virus-mediated transformation, pollen tube pathway and ovary injection method.

In the method of the present invention, the modification of the target sequence can be achieved by only introducing or producing the proteins and the guide RNA in the plant cell, and the modification can be stably inherited, without any need to stably transform the editing system into plants. This avoids the potential off-target effect of the stable editing system and also avoids the integration of the exogenous nucleotide sequence in the plant genome, thereby providing greater biosafety.

In some preferred embodiments, the introduction is carried out in the absence of selection pressure to avoid integration of the exogenous nucleotide sequence into the plant genome.

In some embodiments, the introduction comprises transforming the genome editing system of the present invention into an isolated plant cell or tissue and then regenerating the transformed plant cell or tissue into an intact plant. Preferably, the regeneration is carried out in the absence of selection pressure, i.e., no selection agent for the selection gene on the expression vector is used during tissue culture. Avoiding the use of a selection agent can increase the regeneration efficiency of the plant, obtaining a modified plant free of exogenous nucleotide sequences.

In other embodiments, the genome editing system of the present invention can be transformed into specific parts of an intact plant, such as leaves, shoot tips, pollen tubes, young ears or hypocotyls. This is particularly suitable for the transformation of plants that are difficult to regenerate in tissue culture.

In some embodiments of the invention, the in vitro expressed protein and/or the in vitro transcribed RNA molecule are directly transformed into the plant. The protein and/or RNA molecule is capable of performing genome editing in plant cells and is subsequently degraded by the cell, avoiding integration of the exogenous nucleotide sequence in the plant genome.

Thus, in some embodiments, genetic modification and breeding of plants using the methods of the present invention may result in plants free of integration of exogenous DNA, i.e., transgene-free modified plants.

Plants that can be edited by the system or methods of the invention include monocots and dicots. For example, the plant may be a crop plant such as wheat, rice, corn, soybean, sunflower, sorghum, canola, alfalfa, cotton, barley, millet, sugar cane, tomato, tobacco, tapioca or potato.

In some embodiments of the present invention, the target sequence is associated with a plant trait, such as an agronomic trait, whereby the editing results in a plant having altered traits relative to a wild type plant.

In the present invention, the target sequence to be modified may be located at any position in the genome, for example, in a functional gene such as a protein-encoding gene, or may be, for example, located in a gene expression regulatory region such as a promoter region or an enhancer region, thereby gene functional modification or gene expression modification can be achieved.

In some embodiments of the present invention, the method further comprises obtaining progeny of the genetically modified plant.

In another aspect, the present invention provides a genetically modified plant or a progeny thereof, or a part thereof, wherein the plant is obtained by the method of the invention described above. In some embodiments, the genetically modified plant or a progeny thereof, or a part thereof is transgene-free.

In another aspect, the present invention provides a method of plant breeding comprising crossing a genetically modified first plant obtained by the above method of the present invention with a second plant not containing the genetic modification, thereby the genetic modification is introduced into the second plant.

### Examples

### Example 1 C-to-T conversion mediated by MS2

A plant cytosine base editing system PBE is mainly composed of the following modules: 1) cytosine deaminase used for deamination of cytosine (C) to uracil (U); 2) nCas9 (D10A) used for sgRNA-programmable DNA base editing and promoting endogenous mismatch repair (MMR) pathway; 3) uracil DNA glycosylase inhibitor (UGI) used for inhibiting the activity of uracil glycosylase UDG in vivo to prevent U from converting to an AP site.

Studies have demonstrated that scRNA formed by adding two MS2 hairpins to the 3' end of esgRNA can efficiently mediate CRISPRa in human cells, wherein MS2 is a commonly used RNA aptamer. Therefore, the scRNA vector pOsU3-esgRNA-2×MS2 driven by the OsU3 promoter was first constructed (Fig. 1b). To construct PBEc, a PBE system vector with RNA aptamer recruitment, a T2A "self-cleavge" peptide was used to express multiple protein modules simultaneously, wherein nCas9 (D10A) was fused with or without APOBEC1 or UGI as an RNA-programmable module, and MCP, a MS2 binding protein, was fused with APOBEC1 or UGI as a recruited module, thereby constructing PBEc1 to PBEc5. All PBEc vectors were optimized by crop codons and driven by the *Ubi-1* promoter of maize (Fig. 1c).

In order to screen a highly efficient PBEc vector, the C-to-T efficiency of the PBEc vectors was evaluated by using a BFP-to-GFP reporter system, in which the GFP fluorescence activity required His66 encoded by CAC in BFP to be converted to Tyr66 encoded by TAC. Therefore, a scRNA plasmid esgRNA-2×MS2-BFP targeting this site was constructed, with the base C located at the 4th position at the distal end of PAM. combinations of various PBEc with esgRNA-2×MS2-BFP were transformed into rice protoplasts by way of PEG induction, with PBE and sgRNA-BFP as control groups, GFP as positive control, and untransformed rice protoplasts as negative control. After culture at 22 °C for 36 hours, GFP fluorescent activity of each treatment group was detected by flow cytometry. The replicated results of three experiments showed that the GFP fluorescent activity of PBEc1 to PBEc5 was 0.67-10.80%, among which, the fluorescent activity of PBEc4 with MCP-APOBEC1-UG1 recruited module was the highest, followed by PBEc5 with MCP-UGI-APOBEC1 recruited module, which was 2.87 times and 1.21 times that of PBE and sgRNA-BFP control groups, respectively (Fig. 1d). Both PBEc1 and PBEc2 had the recruited module MCP-APOBEC1, and their efficiencies were comparable to that of PBE control group (Fig. 1d). Although MCP binded to the hairpin structure of MS2 as a dimer, but when the recruited module was MCP-UGI (PBEc3), the efficiency of C-to-T declined dramatically (Fig. 1d).

To verify the C-to-T activity of PBEc vectors on endogenous target sites, six rice endogenous target sites were constructed on the pOsU3-esgRNA-2×MS2-BFP or pOsU3-sgRNA vector (Table 1). scRNA vectors containing target sites were co-transformed into rice protoplasts combined with PBEc1 to PBEc5, respectively, and sgRNA vectors containing target sites were co-transformed into rice protoplasts combined with PBE and Cas9, respectively, as control groups. After culture at 22°C for 60 hours, the rice protoplast DNA was extracted, followed by amplicon NGS sequencing. The results showed that PBEc vectors paired with MS2 and MCP had the same C₃-C₉ editing window as PBE (Fig. 1e). The editing efficiencies at C₃-C₉ of the endogenous target sites by using the tested five PBEc vectors ranged from 0.13% to 11.73% (Fig. 1e), with PBEc4 the most efficient and its activity being 3.62 times that of PBE at four target sites (*OsACC-T1, OsDEP1-T2, OsEV and OsOD*)*,* and slightly lower than that of PBE at two target sites (*OsCDC48* and *OsDEP1*-T1). In addition, nPBEc4 had a high product-editing purity with unobvious non-target products (< 0.04%); the resulting indel efficiency (0.04~0.29%) was identical to that of untreated rice protoplasts (0.01~0.32%), but much less than that of Cas9 (4.82~11.75%) (Fig. 5). In conclusion, the scRNA-programmable nCas9 (D10A) complex can improve the C-to-T base editing activity by recruiting APOBEC1 and UGI at the same time. Therefore, the PBEc4 vector structure was used as the object for further research.

**Table 1. sgRNA target sequences for comparing activities of PBEc and PABEc**

| Target name | Target sequence (5'-3') ^{a} |
|---|---|
| BFP | ACCCACGGCGTGCAGTGCTT**CAG** |
| mGFP | GCACTACACGCCGTAGGTGA**AGG** |
| *OsACC-T1* | TACTAGTCACACTTGCACTG**TGG** |
| *OsALS-* T1 | CCCAAGTGGGGGCGCATTCA**AGG** |
| *OsCDC48* | TAGCACCCATGACAATGACA**TGG** |
| *OsDEP1-T1* | AGCACATGAGAGAACAATAT**TGG** |
| *OsDEP1-*T2 | AGACAAGCTTGGCCCTCTTT**GGG** |
| *OsNRT1.1B* | ACTAGATATCTAAACCATTA**AGG** |
| *OsEV* | CACACACACACTAGTACCTC**TGG** |
| *OsOD* | ACACACACACTAGTACCTCT**GGG** |

| | |
|---|---|
| ^{a} PAM motif was shown in bold and underlined. | |

### Example 2 Designing various scRNAs for mediating C-to-T conversion

In order to develop a multiplex recruitment system and create a variety of scRNAs with different RNA aptamers, PBEc6, PBEc7 and PBEc8 were constructed by replacing MCP in PBEc4 with PCP, N22p and Com, which recognize the viral RNA hairpins PP7, boxB and com, respectively (Figure 2a). It has been reported that sgRNA 2.0, which was formed by embedding the MS2 hairpin on the tetraloop and stem loop 2 (secondary structure) of sgRNA, was more efficient than sgRNA-2×MS2, a scRNA form formed by linking two MS2 hairpins at the 3' end of sgRNA. Therefore, sgRNA2.0 to sgRNA4.0 were constructed by embedding a MS2 hairpin on the tetraloop and stem loop 2 of sgRNA or esgRNA containing one A-to-U or C-to-G flip and extending hairpin, respectively (Figs. 2b, 16 and 17). Using the same construction strategy, sgRNA7-1, sgRNA7-2 and sgRNAB.0 were constructed, which contain two PP7 hairpin variants and boxB hairpins, respectively (Fig. 2b, 16 and 17). At the same time, a variety of scRNAs were constructed by using the strategy of linking one or two RNA aptamer (including MS2, PP7, boxB, com) hairpins at the 3'-end (Figs. 2b, 16 and 17). For recruiting more APOBEC1-UGI modules, sgRNA4.0 with MS2 hairpins was constructed by combining with the above two strategies (Figs. 2b, 16 and 17).

In order to compare the activities among different scRNAs with corresponding PBEcs, the efficiency was evaluated by using the BFP-GFP reporter system in rice protoplasts. BFP-sgRNA or BFP-esgRNA and the PBE vector were used as control groups. Unlike the reported conclusions, the C-to-T editing efficiencies meditated by all sgRNA2.0-constructed scRNAs (comprising MS2, PP7 and boxB) in the reporter system were all very low (0.07~0.43%) (Fig. 2c). Conversely, both scRNA containing two or three RNA aptamer hairpins at the 3'-end and esgRNA-1 ×com containing one hairpin can mediate powerful C-to-T editing efficiencies (1.83-8.83%) (Fig. 2c). Among them, the C-to-T editing efficiencies in the reporter system obtained by the transformation combinations of PBEc4 with esgRNA-2×MS2,esgRNA-3×MS2 or sgRNA4.0 and the transformation combination of PBEc8 with esgRNA-2×com were 7.47%, 8.00%, 8.83%, and 6.90%, respectively, all of which were higher than that obtained by the transformation combination of PBE with esgRNA (6.03%) (Fig. 2c). The activities for the two scRNA conformations was inconsistent with the reported conclusions due to that a double-stranded linker was used herein between RNA aptamer hairpins at the 3'-end of scRNA, thus improving the conformational stability of the 3'-end with multiple hairpins of scRNA.

To evaluate the C-to-T editing activities at rice endogenous target sites mediated by the above highly efficient SCRNAs (esgRNA-2×MS2, esgRNA-3×MS2, sgRNA4.0, esgRNA-2×com), five endogenous target sites were constructed on the four scRNA vectors, which were co-transfected into rice protoplasts combined with PBEc4 and PBEc8, respectively. sgRNA or esgRNA vectors containing target sites were co-transformed into rice protoplasts combined with PBE and Cas9, respectively, as control groups. After culture at 22°C for 60 hours, the rice protoplast DNA was extracted, followed by amplicon NGS sequencing. The results showed that at the C₃-C₉ editing window of five target sites *(OsACC-T1, OsDEP1-T1, OsDEP1-T2, OsEV,* and *OsOD*)*,* the C-to-T base editing activities mediated by esgRNA (average 7.96%), esgRNA-2×MS2 (average 18.04%), esgRNA-3×MS2 (average 14.96%) and esgRNA-2×com (average 11.13%) were all higher than those mediated by sgRNA (average 4.82%) and sgRNA4.0 (average 4.78%). Among these scRNAs, the efficiencies of C-to-T base editing mediated by esgRNA-2×MS2, esgRNA-3×MS2 and esgRNA-2×com were 2.31-3.75 times higher than that mediated by sgRNA (Fig. 2d, Fig. 6), and all of which had the same main single base window C₃-C₉ (Fig. 2d). At the same time, different scRNAs in rice protoplasts also yielded higher product purity (> 99.68%) (Fig. 7a) and lower indel reads (< 0.56%), far less than that with Cas9 (<21.21%) (Fig. 7b).

In addition, in order to develop an efficient PBE system with a narrow editing window, APOBEC1 of PBE and PBEc4 was replaced by APOBEC1 variants YE1, YE2, EE and YEE with reduced catalytic activity (Fig. 8a, Fig.b). The C-to-T base editing window and activity with these variants under PBE and PBEc4 conformations were tested by using two target sites *OsEV* and *OsOD* in rice protoplasts. The results of amplicon sequencing showed that the combination of YE1-PBEc4, EE-PBEc4 or YEE-PBEc4 with esgRNA-2×MS2 each enabled the improvement of C-to-T base editing activity, and the activity at the middle of the editing window (C₅ of *OsEV* and C₆ of *OsOD*) was about 1.37-1.78 times those resulting from these variants under PBE conformation (Fig. 2e, Fig. 8c). Among them, EE-PBEc4 yielded low subordinate editing products while keeping a narrow window (Fig. 2e, Fig. 8c), indicating that PBE with a narrow window can enhance its editing activity by way of scRNA recruitment.

In conclusion, the incorporation of different RNA aptamers onto sgRNA provides an effective solution for employing RNA programmable nCas9 (D10A) multiplex recruitment in plants. In addition, esgRNA-2×MS2, esgRNA-3×MS2 and esgRNA-2×com can be chosen as candidates for mediating CBE function in the multiplex genome editing system.

### Example 3 Optimizing vector and scRNA for mediating C-to-T conversion

PABE-7, a plant adenine single-base editor, mainly consists of the following modules: a heterodimer composed of wild-type adenine deaminase ecTadA and artificially evolved deoxyadenine deaminase ecTadA7.10, nCas9 (D10A) consistent with the PBE system, and three copies of SV40 NLS at the C-terminal of nCas9 (D10A). To modify PABE-7 to the conformation for RNA aptamer recruitment, PABEc1 for esgRNA-2×MS2 recruitment was first constructed based on PBEc4 (Fig. 3a). PABEcl-mediated A-to-G base editing activity was tested using a mGFP reporter system, in which the conversion of A-to-G on the non-coding strand enabled the conversion of stop codon TAG on the coding strand to CAG (Gln69), thus generating GFP fluorescence reporter activity. PABEc1, Ubi-mGFP and mGFP-esgRNA-2×MS2 were co-transformed into rice protoplasts, after culture at 22 °C for 24 hours, GFP fluorescence activity thereof was detected by flow cytometry. The results showed that, unlike the combination of PBEc4 and esgRNA-2×MS2 characterized by improving C-to-T activity, the A-to-G activity of PABEc1 in the mGFP reporter system (1.73%) was much lower than that of PABE-2 (7.03%) commonly used in human cells (Fig. 3b). Since the linker with 32 amino acids between ecTadA^{∗} variant and nCas9 provided higher efficiency than XTEN linker with 16 amino acids, PABEc2, as well as PABEc3 with MCP at C-terminal, was constructed by replacing the XTEN linker of PABEc1 with the linker having 32 amino acids ((SGGS)₂-XTEN-(SGGS)₂) (Fig. 3a). In the mGFP reporter system, the activities of PABEc2 and PABEc3 were 7.23% and 8.03%, respectively, which were slightly higher than that of PABE-2, but still lower than the efficiency of the combination of PABE-7 and esgRNA (14.40%) (Fig. 3b). To evaluate the effectiveness of a SAM-like construct form in improving the A-to-G activity, PABEc4 was constructed by fusing ecTadA-ecTadA7.10 heterodimer to the C-terminal of nCas9 (D10A) in PABEc3 (Fig. 3a). However, in the mGFP reporter system, the A-to-G base editing activity of PABEc4 was 10.60%, which was still lower than that of PABE-7 combined with esgRNA (Fig. 3b). The results suggested that improving the A-to-G base editing activity of PABEc in plants by optimizing its conformation was restricted. In conclusion, PABEc3 conformation was used for further development of multiple systems, and other RNA aptamers were attempted to enhance the activity of this conformation.

PABEc5, PABEc6 and PABEc7 were constructed by replacing MCP at C-terminal of PABEc3 with PCP, N22p and Com, which were used to identify RNA hairpins PP7, boxB and com, respectively (Fig. 3c). The combinations of corresponding scRNAs with PABEc3, PABEc5, PABEc6 or PABEc7 were tested using the mGFP reporter system. Among them, the A-to-G activity of the combination of PABEc6 and esgRNA-2×boxB in the reporter system was 26.53%, which was slightly higher than that of PABE-7 combined with esgRNA (25.57%) (Fig. 3d). Among other types of RNA aptamers, the combination with the highest A-to-G activity in the reporter system was as follows in turn: PABEc3 combined with esgRNA-2×MS2+f6, PABEc5 combined with esgRNA-1×PP7-1, PABEc7 combined with esgRNA-2×com, with the efficiency of 18.07%, 21.03% and 22.47%, respectively, which were all lower than that of PABE-7 combnined with esgRNA (Fig. 3d). At the same time, PABEc8, PABEc9 and PABEc10 based on PABEc2 conformation were also constructed (Fig. 9a) by using the PCP, N22p or Com binding protein at the N-terminal of adenine deaminase, respectively. However, the efficiencies of all tested combinations in the mGFP reporter system (< 21.23%) were lower than that of PABE-2 combined with sgRNA (22.87%) (Fig. 9b). Like PBEc employing scRNA containing RNA hairpins on the tetraloop and stem loop 2, PABEc and these scRNAs also mediated lower GFP fluorescence signals (Fig. 3d). Therefore, conversions of C-to-T and A-to-G mediated by scRNA with RNA hairpins at 3'-end were more efficient than those mediated by scRNAs with RNA hairpins on the tetraloop and stem loop 2.

To evaluate the efficiency of PABEc-mediated A-to-G base editing in rice endogenous genes, esgRNA-2×MS2, esgRNA-2×MS2+f6, esgRNA-1×PP7-1, esgRNA-2×boxB and esgRNA-2×com were employed to construct six endogenous target vectors, respectively, which were co-transformed into rice protoplasts combined with corresponding PABEcs (Table 1). The combination of PABE-2 and sgRNA and the combination of PABE-7 and esgRNA were used as control groups. Among the tested transformation combinations of PABEcs and scRNAs, the highest A-to-G base editing efficiency was mediated by the combination of PABEc6 and esgRNA-2×boxB, with an average efficiency of 4.65% at the main editing window A₄-A₈, which was comparable to that mediated by the combination of PABE-2 and sgRNA (average 4.78%) (Fig. 3e, Fig. 10a). As N22p has the shortest length of 33 amino acids among the binding proteins tested, it was speculated that the activity of the heterodimer ecTadA-ecTadA7.10 was affected not only by the position but also the length of the binding protein. Similarly, the combination of PABEc6 and esgRNA-2×boxB also gave higher product purity (average 99.76%) in rice protoplasts (Fig. 10b), with its indel reads of 0.04-0.38%, which was consistent with that of the untreated control group (0.05-0.40%) and much less than that of Cas9 (10.94-23.84%) (Fig. 10c). Therefore, esgRNA-2×boxB was chosen as the scRNA for mediating ABE in the multiplex genome editing system.

### Example 4 Cas9 nickase-based multiplex genome editing system

The successful application of scRNA-mediated CBE or ABE in rice protoplasts provided a basis for further development of multiplex genome editing system using the nCas9 (D10A) platform for simultaneous editing. To harness the function of nCas9 (D10A) to mediate multiplex genome editing, SWISSv1.1 was integrated based on PBEc4, simultaneously expressed one esgRNA-2×MS2 and one paired sgRNAs, to generate simultaneous cytosine base editing and paired nCas9-mediated DSB at different target sites (Fig. 4a). Two groups of sgRNA were tested (Table 2), and multiple sgRNAs of each group were assembled on the same vector and driven by *OsU3* or *TαU6* (Fig. 11). In rice protoplasts, the C-to-T editing activity at the editing window C₃-C₉ of the tested target site was 0.33~31.32%, while the indel efficiency at another target site was 1.74-2.52% (Fig. 4a). Employing the same strategy, SWISSv1.2 was integrated based on PABEc6, esgRNA-2×boxB, and paired sgRNAs (Fig. 4b). In the two tested groups of rice protoplasts, the A-to-G efficiency reached 2.85%, while the indels efficiency at another target site reached 2.49% (Fig. 4b). In addition, the NGS sequencing results showed that, in SWISSv1.1 and SWISSv1.2, at least 79% of indels mutant sequence reads induced by paired nCas9 (D10A) were deletions (Fig. 12). The above results indicated that scRNA-mediated PBE and PABE systems each can realize a dual function of base editing and indels by using multiple sgRNAs, demonstrating that paired nCas9 (D10A) can generate indels when employing PBEc4 and PABEc6.

To test the ability of scRNA-mediated base editing for carrying out a dual function of CBE and ABE at different target sites simultaneously, a MGE vector was constructed based on nCas9 (D10A), MCP-APOBEC1-UGI, and ecTadA-ecTadA7.10-N22p, which were simultaneously expressed by the *Ubi-1* promoter and a T2A "self-cleave" peptide (Fig. 13a). SWISSv2 (Fig. 4c) was formed by integrating EsgRNA-2×MS2 driven by the *TaU6*promoter for C-to-T editing, esgRNA-2×boxB driven by the *OsU3* promoter for A-to-G editing (Fig. 13B) with MGE. Two groups of target sites were tested in rice protoplasts (Table 2). The results showed that SWISSv2 could generate C-to-T (<13.19%) base editing at one target site, while generating A-to-G (<4.27%) base editing at another target site. Paired sgRNA were added to SWISSv2 to obtain SWISSv3 (Fig. 4d). Still, two groups of target sites were tested in rice protoplasts (Table 2, Fig. 13c). The amplicon NGS sequencing results showed that SWISSv3, as a multiplex integrated programmable genome editing system, can simultaneously achieve tri-functional editing at different target sites: C-to-T (<11.68%), A-to-G (<2.64%) and indels (<2.22%) (Fig. 4e). In conclusion, SWISSv3 provides an alternative scheme for gene superposition and heritable modification in plants.

**Table 2 sgRNA target sequences for testing SWISSv1.1, SWISSv1.2, SWISSv2 and SWISSv3 in rice protoplasts**

| Genome editing type | Target name | Target sequence (5'-3') ^{a} |
|---|---|---|
| CBE | *OsALS-T2* | CGCGTCCATGGAGATCCACC**AGG** |
| | *OsBADH2* | CGCGCAATCGCGGCCAAGGT**AGG** |
| | *OsCDC48* | TAGCACCCATGACAATGACA**TGG** |
| | *OsGL2* | CTTCCACAGGCTTTCTTGAA**CGG** |
| | *OsDEP1-NGA* | GTGAAGCTCATCCTACAAAA**GGA** |
| | *OsCDC48-NGA* | CCATGACAATGACATGGGAA**CGA** |
| ABE | *OsACC-T2* | CATAGCACTCAATGCGGTCT**GGG** |
| | *OsDEP1-T1* | AGCACATGAGAGAACAATAT**TGG** |
| | *OsNRT1.1A* | GTGGACGCTGGCGACGCTGA**CGG** |
| | *OsAAT-*NGT | TACAACAAGGATCCCAGCCC**CGT** |
| | *ODEP1*-NGT | GCACATGAGAGAACAATATT**GGT** |
| DSB ^{b} | *OsBADH2-*Indels-sgL | CTGCCGCTGCGGGATCGCCGT**GG** |
| | *OsBADH2*-Indels-sgR | GAGTGGCGCGCCCCCGCGCT**CGG** |
| | *OsNRT1.1A*-Indels-sgL | TCGTCGGAGGCGAGTGGTGG**TGG** |
| | *OsNRT1.1A*-Indels-sgR | CTCTCACCTGCGTTTGTCGC**CGG** |
| | *OsNRT1.1B*-Indels-sgL | GTTTAGATATCTAGTAGTGC**TGG** |
| | *OsNRT1.1B*-Indels-sgR | GGTCAAATCAAGTTATTTTT**AGG** |
| | *OsCDC48-*Indels-sgL | AATGACATGGGAACGAGCTT**TGA** |
| | *OsCDC48*-Indels-sgR | GTGCTACAAACCGGCCAAAC**AGT** |

| | | |
|---|---|---|
| ^{a} PAM motif was shown in bold and underlined. ^{b} paired sgRNAs were designed according to the following rules: (1) in a PAM-out orientation; (2) the distance between nicking sites was 40-68 bp. | | |

### Example 5 Multiplex editing in rice plants

To verify the editing ability of SWISSv3 in rice plants, a multi-sgRNA vector targeting *OsALS, OsACC* and *OsBADH2* was constructed and assembled together with MGE into a binary vector pCAMBIA1300 (Fig. 14a). Mutation sites in regenerated rice plants were detected by T7E1 and Sanger sequencing (Fig. 14b). The results showed that the efficiencies of CBE, ABE and indels were 25.45%, 16.36% and 52.73%, respectively (Table 3). More importantly, four plants contained a triple mutant with simultaneous CBE, ABE and indels at three different target sites, with an efficiency of 7.27%; up to 12.73% of the regenerated plants were a double mutant; SWISSv3 can also produce a single mutant at three sites (Table 4). In conclusion, SWISSv3, as a tri-functional integrated programmable genome editing system with scRNAs in plants, will be beneficial to molecular breeding in crops.

**Table 3. Mutation frequencies induced by SWISSv3 in T0 rice plants**

| Genome editing type | Target name | Number of transgenic rice strains | Mutant strain^{a} | Mutant genotype ^{b} | heterozygous/ homozygous |
|---|---|---|---|---|---|
| CBE | *OsALS-T2* | 55 | 14 (25.45%) | 7 C₆>T₆; 2 C₆>G₆; 4 C₆C₇>T₆T₇; 1 | 14/0 |
| | | | C₆C₇C₁₆>T₆T₇T₁₆ | | |
| ABE | *OsACC-T2* | 9 (16.36%) | 7 A₄>G₄; 2 A₇>G₇ | 9/0 | |
| Indels | *OsBADH2-I* ndels | 29 (52.73%) | 22 deletions; 7 insertions | 19/10 | |

| | | | | | |
|---|---|---|---|---|---|
| ^{a} The number of T0 strains (rice) carrying the observed mutation over the total number of T0 transgenic rice strains analyzed. ^{b} The genotypes of indels were analyzed by online tools DSDecodeM (reference) and TIDE (reference). | | | | | |

**Table 4. Multiplex genome editing in T0 rice plants using SWISSv3**

| Edit event | Mutant target | Mutant genotype |
|---|---|---|
| Single | *OsALS-*T2 | 2 (3.64%, 2 A₄>G₄) |
| | *OsACC-*T2 | 2 (3.64%, 1 C₆>T₆; 1 C₆C₇>T₆T₇) |
| | *OsBADH2-*Indels | 16 (29.09%, 12 deletions; 4 insertions) |
| double | *OsALS-T2*/*OsACC-*T2 | 1 (1.82%, 1 C₆>T₆+A₄>G₄) |
| | *OsALS-T2*/*OsBADH2-*Indels | 7 (12.73%, 2 C₆>T₆+deletions; 1 C₆>T₆+insertion; 1 C₆>G₆+deletion; 2 C₆C₇>T₆T₇+deletions; 1 C₆C₇C₁₆>T₆T₇T₁₆+deletion) |
| | *OsACC-T2*/*OsBADH2-*Indels | 2 (3.64%, 1 A₄>G₄+deletion; 1 A₇>G₇+deletion) |
| Three types of editing | *OsALS-*T2/*OsACC-T2*/*OsBADH2-*Indels | 4 (7.27%, 1 C₆>T₆+A₄>G₄+ deletion; 1 C₆>G₆+ A₄>G₄+ insertion; 1 C₆>T₆+A₇>G₇+ deletion; 1 C₆C₇>T₆T₇+ A₄>G₄+ deletion) |
| Total number | N.A.^{a} | 55 |

| | | |
|---|---|---|
| ^{a} N.A. stands for unavailable. | | |

### Example 6 Off-target analysis of SWISS system

In SWISSv2 and SWISSv3, T2Awere used for simultaneous expression of multiple modules, and it was speculated that the "self-cleavge" efficiency of T2A would affect the product purity at the CBE or ABE target site (Fig. 15a). As shown in Fig. 15a, the T2A-mediated "self-cleavge" wad carried out by forming a glycine-proline peptide bond at the C-terminal through ribosome skipping of T2A, and the position of T2A would affect the simultaneous expression level of multiple modules. In MGE, successful skipping will produce three independent target proteins, but failure of skipping will produce undesired fusion proteins, especially MCP-APOBEC1-UGI-T2A-ecTadA-ecTadA7.10-N22 fusion protein, which can be recruited by both esgRNA-2×MS2 and esgRNA-2×boxB, resulting in undesired C base editing at the ABE target site or undesired A base editing at the CBE target site, thereby resulting in undesired off-target. The amplicon NGS sequencing results of rice protoplasts employing SWISSv2 and SWISSv3 were analyzed, and the efficiencies of cytosine editing at the ABE target site and adenine editing at the CBE target site were checked. The results showed that both of them suffered from undesired editing, the efficiencies of undesired C-to-T editing and A-to-G editing were lower than 0.90% and 0.19%, respectively, but still higher than that of the untreated control group (C-to-T, <0.07%; A-to-G, <0.04%) (Fig. 15b). At the same time, the results suggested that theres was a need for employing a more efficient strategy for co-expressing multiple modules.

Potential off-target sites with a mismatch of less than or equal to 3 nt were further searched using Cas-OFFinder at the whole genome level, which were then sequenced. The results showed that no off-target event was found at all potential off-target sites (Table 5). Due to the integration of cytosine deaminase and adenine deaminase into the SWISS system, potential unpredictable DNA and RNA off-target may occur, so it is necessary to employ highly efficient and specific deaminase variants for further solving this problem.

**Table 5. Analysis of potential off-target sites on OsALS-T2, OsACC-T2, OsBADH2-Indels-sgL and OsBADH2-Indels-sgR in the triple mutant.**

| Target name | Potential off-target site | Sequence (5'-3') ^{a} | Mism atch numb er | Target gene locus | Editing |
|---|---|---|---|---|---|
| *OsALS-T2* | On-target | CGCGTCCATGGAGA TCCACCAGG | 0 | LOC_Oₛ02g30630.1 | C-to-T |
| | Off-targetl | CGCGTCaATGGAGA TCCACCAGG | 1 | LOC_Oₛ04g32010.1 | NO |
| | Off-target2 | CGCGTCgATGGAGA TCCACCAGG | 1 | LOC_Os04g31960.1 | NO |
| | Off-target3 | CGCGTCCAaGGAGtT CCAgCAGG | 3 | LOC_Oₛ10g08540.1 | NO |
| | Off-target4 | CGCGTCCAaGGAGtT CCAgCAGG | 3 | LOC_Oₛ10g08540.1 | NO |
| *OsACC-T2* | On-target | CATAGCACTCAATG CGGTCTGGG | 0 | LOC_Os05g22940.1 | A-to-G |
| | Off-target | N.A. ^{b} | 1-3 | N.A.^{b} | N.A.^{b} |
| *OsBADH2-*Indels-sgL | On-target | CTGCCGCTGCGGGA TCGCCGTGG | 0 | LOC_Oₛ08g32870.1 | Indels |
| | Off-target1 | CTGCCGCTGCGGcgT CGgCGAGG | 3 | LOC_Oₛ03g52640.1 | NO |
| | Off-target2 | CTGCCGCTcCtGGAa CGCCGAGG | 3 | LOC_Oₛ01g07160. I | NO |
| | Off-target3 | CgGCCGCaGCGGGA gCGCCGGGG | 3 | LOC_Os02g35190.1 | NO |
| | Off-target4 | CTGCCGCTGCtGccT CGCCGTGG | 3 | LOC_Os02g43220.1 | NO |
| | Off-target5 | CcGCCGCgGCGGGtT CGCCGCGG | 3 | LOC_Os10g37770.1 | NO |
| | Off-target6 | CTGCtGCTGCGccAT CGCCGGGG | 3 | LOC_Os06g46440.1 | NO |
| | Off-target7 | CTGgCGCTGCGGGA gCGgCGTGG | 3 | LOC_0s11g03550.1 | NO |
| | Off-target8 | CTGCaGCcGCGGGAa CGCCGCGG | 3 | LOC_Os11g42240.1 | NO |
| *OsBADH2-*Indels-sgR | On-target | GAGTGGCGCGCCCC CGCGCTCGG | 0 | LOC_Os08g32870.1 | Indels |
| | Off-targetl | GAGTGGCGCGCCCg CGCGgTTGG | 2 | LOC_Os04g18380.1 | NO |
| | Off-target2 | GAGTGGCGCGCCtC CaCGCTCGG | 2 | LOC_Os01g22370.1 | NO |
| | Off-target3 | GAGTGGCGCGCCCg CGCGgTGGG | 2 | LOC_Os01g64256.1 | NO |
| | Off-target4 | GAGTGGCGCttCCCC GCGCaCGG | 2 | LOC_0s06g12810.1 | NO |

| | | | | | |
|---|---|---|---|---|---|
| ^{a} PAM motif was shown in bold and underlined; ^{b}N.A. standed for unavailable. | | | | | |

The multiplex genome editing system using multiple sgRNAs can be classified into two ways: one is to perform the same type of genome editing at different target sites; the other is to perform different types of genome editing at different target sites as provided herein. So far, it is the first time that the SWISS system developed herein can use a programmable Cas protein to mediate multiplex different types of genome editing in plants simultaneously. Although this multiplex editing can be carried out by CRISPR/Cas cognate proteins, the vector for multiple cognate proteins would be larger in size, which is not conducive to genetic transformation mediated by a gene gun, and the requirement for PAM would also be more restricted. However, the SWISS system that uses only one kind of nCas9 (D10A) can alleviate the problems caused by the above two shortcomings, especially, the use of Cas9-NG PAM variants would further expand the editing range of SWISS (Fig. 4e, Table 2).

RNA polymerase type III promoters OsU3 and TaU6 are used herein to express multiple sgRNAs, and other multiple-sgRNA strategies, such as using Csy4 RNA ribonuclease or ribozyme to produce multiple sgRNAs, can also be employed to further optimize the SWISS system. Since the average C-to-T activity of scRNA-recruited constructs was higher than that of PBE, without accompanied by a wider base editing window as well, this strategy could be used to improve the editing activity of narrow-window cytidine deaminase variants. Although the A-to-G activity of scRNA-recruited constructs was just comparable to that of PABE-2, it is enough to mediate SWISSv3 to obtain rice A-to-G mutants. At the same time, unlike PBEc constructs, using different RNA aptamers can not improve the efficiency of PABEc constructs, which also means that the space for optimizing PABEc is limited, and it is necessary to develop more efficient adenine deaminases.

Of course, the dual-functional SWISSv1.1 and SWISSv1.2 systems can also be implemented by employing PBE and PABE combined with multiple sgRNAs. However, the RNA aptamer recruitment strategy herein provides another alternative method, especially the use of nCas9 (DlOA)-overexpressing plant for multiplex genome editing, which is advantageous. Therefore, nCas9 (D10A)-overexpressing rice may be constructed in the future as a development platform, through which, it only needs to transform multiple sgRNAs and base editing recruitment modules, and the multiplex editing function of SWISS can be carried out through secondary transformation, which may also reduce the undesired off-target events, and is beneficial to molecular design breeding in crops. In addition, a quad-functional CRISPR system can be implemented by optimizing the third scRNA, constructing a truncated spacer sequence (14-15 nt), and recruiting an epigenetic modifier, a gene regulation inhibitor or activator, or a fluorescent protein. The SWISS system can also adopt random and multiple sgRNA strategies for directed evolution of plant endogenous genes, as well as applications beyond plants, such as changing cell fate or metabolic regulation pathways.

## Claims

1. A genome editing system for multiplex editing in a plant, especially a genetically modified crop, comprising:
i) a CRISPR nickase and/or an expression construct containing a nucleotide sequence encoding the CRISPR nickase; and
ii) one or more or all items selected from the group consisting of:
ii-1) a first scRNA targeting a first target region in the plant genome and/or an expression construct containing a nucleotide sequence encoding the first scRNA, wherein the first scRNA comprises at least one first RNA aptamer; and, a first fusion protein and/or an expression construct containing a nucleotide sequence encoding the first fusion protein, wherein the first fusion protein comprises a first RNA aptamer-specific binding protein and a cytosine deamination domain;
ii-2) a second scRNA targeting a second target region in the plant genome and/or an expression construct containing a nucleotide sequence encoding the second scRNA, wherein the second scRNA comprises at least one second RNA aptamer; and, a second fusion protein and/or an expression construct containing a nucleotide sequence encoding the second fusion protein, wherein the second fusion protein comprises a second RNA aptamer-specific binding protein and an adenine deamination domain;
ii-3) paired gRNAs targeting a third target region in the plant genome and/or an expression construct containing nucleotide sequences encoding the paired gRNAs, wherein the paired gRNAs target different strands of DNA in the third target region, respectively.

2. The system according to claim 1, wherein the CRISPR nickase is a Ca9 nickase, for example, a Ca9 nickase comprising the amino acid sequence shown in SEQ ID NO: 25 or 48.

3. The system according to claim 1 or 2, wherein the paired gRNAs comprise the nucleotide sequence shown in SEQ ID NO: 3 or SEQ ID NO: 4.

4. The system according to any one of claims 1 to 3, wherein the RNA aptamer is selected from MS2, PP7, boxB and com.

5. The system according to any one of claims 1 to 4, wherein the RNA aptamer-specific binding protein is selected from MCP, PCP, N22p, and COM.

6. The system according to any one of claims 1 to 5, wherein the scRNA comprises two or more RNA aptamers.

7. The system according to any one of claims 1 to 6, wherein the scRNA comprises the nucleotide sequence shown in one of SEQ ID NOs: 5-24.

8. The system according to any one of claims 1 to 7, wherein the first scRNA comprises the nucleotide sequence shown in SEQ ID NO: 13 or 15.

9. The system according to claim 8, the first RNA aptamer-specific binding protein comprises the amino acid sequence shown in SEQ ID NO: 34.

10. The system according to any one of claims 1 to 7, wherein the first scRNA comprises the nucleotide sequence shown in SEQ ID NO: 24.

11. The system according to claim 10, wherein the first RNA aptamer-specific binding protein comprises the amino acid sequence shown in SEQ ID NO: 37.

12. The system according to any one of claims 1 to 11, wherein the second scan comprises the nucleotide sequence shown in SEQ ID NO: 22.

13. The system according to claim 12, wherein the second RNA aptamer-specific binding protein comprises the amino acid sequence shown in SEQ ID NO: 36.

14. the system according to any one of claims 1 to 13, wherein the cytosine deaminase is selected from APOBEC1 deaminase, activation-induced cytidine deaminase (AID), APOBEC3G, CDA1, human APOBEC3A deaminase, or functional variants thereof.

15. The system according to claim 14, wherein the cytosine deaminase is APOBEC1 deaminase or its functional variant.

16. The system according to claim 15, wherein the cytosine deaminase comprises the amino acid sequence shown in one of SEQ ID NOs: 26-30.

17. The system according to any one of claims 1 to 16, wherein the first RNA aptamer-specific binding protein is located at the N-terminal of the cytosine deamination domain.

18. The system according to any one of claims 1 to 17, wherein the first RNA aptamer-specific binding protein is fused with the cytosine deamination domain via a linker.

19. The system according to any one of claims 1 to 18, wherein the first fusion protein further comprises uracil DNA glycosylase inhibitor (UGI), for example, the UGI comprises the amino acid sequence shown in SEQ ID NO: 31.

20. The system according to any one of claims 1 to 19, wherein the adenine deamination domain comprises at least one DNA-dependent adenine deaminase polypeptide.

21. The system according to claim 20, wherein the DNA-dependent adenine deaminase is a variant of Escherichia coli tRNA adenine deaminase TadA (ecTadA), for example, the DNA-dependent adenine deaminase comprises the amino acid sequence shown in SEQ ID NO: 33.

22. The system according to claim 21, wherein the adenine deamination domain further comprises a corresponding wild-type Escherichia coli tRNA adenine deaminase TadA fused with the DNA-dependent variant of the Escherichia coli tRNA adenine deaminase TadA, for example, the wild-type Escherichia coli tRNA adenine deaminase TadA comprises the amino acid sequence shown in SEQ ID NO: 32.

23. The system according to claim 22, wherein the DNA-dependent variant of the Escherichia coli tRNA adenine deaminase TadA is fused to the C-terminal of the corresponding wild-type Escherichia coli tRNA adenine deaminase TadA, preferably by a linker.

24. The system according to any one of claims 1 to 23, wherein the second RNA aptamer-specific binding protein is located at the C-terminal of the adenine deamination domain.

25. The system according to any one of claims 1 to 24, wherein the second RNA aptamer-specific binding protein is fused with the adenine deamination domain via a linker.

26. The system according to any one of claims 1 to 25, wherein the CRISPR nickase, the first fusion protein and/or the second fusion protein further comprise a nuclear localization sequence (NLS).

27. The system according to any one of claims 1 to 26, wherein the CRISPR nickase, the first fusion protein and/or the second fusion protein are interlinked by a "self-cleavage" peptide.

28. A method for generating a genetically modified plant, such as a genetically modified crop, comprising introducing the genome editing system according to any one of claims 1 to 27 into the plant.

29. The method according to claim 28, wherein i) and ii-1) of the system are co-introduced into the plant, thereby carrying out C-to-T editing at the first target site.

30. The method according to claim 28, wherein i), ii-1) and ii-2) of the system are co-introduced into the plant, thereby carrying out C-to-T editing at the first target site, and A-to-G editing at the second target site.

31. The method according to claim 28, wherein i), ii-2) and ii-3) of the system are co-introduced into the plant, thereby carrying out A-to-G editing at the second target site, and deletion mutation at the third target site.

32. The method according to claim 28, wherein i), ii-1) and ii-3) of the system are co-introduced into the plant, thereby carrying out C-to-T editing at the first target site, and deletion mutation at the third target site.

33. The method according to claim 28, wherein i), ii-1), ii-2) and ii-3) of the system are co-introduced into the plant, thereby carrying out C-to-T editing at the first target site, A-to-G editing at the second target site, and deletion mutation at the third target site.

34. The method according to claims 28-33, wherein i), ii-1), ii-2) and ii-3) and combinations thereof in the system are introduced into the plant at the same time, such as in the same vector or in one transformation.

35. The method according to claim 28, comprising:
c) introducing i) of the system into the plant to obtain a transgenic plant stably expressing the CRSPR nickase;
d) introducing i-1), ii-2) ii-3) or any combination thereof of the genome editing system into the transgenic plant obtained in step a).

36. The method according to any one of claims 28-35, wherein the plant includes monocotyledon and dicotyledon, for example, the plant is a crop such as wheat, rice, corn, soybean, sunflower, sorghum, rape, alfalfa, cotton, barley, millet, sugarcane, tomato, tobacco, cassava, or potato.
